# EUROPÄISCHE PATENTANMELDUNG

(11) **EP 2 845 582 A1**
(43) Veröffentlichungstag der Anmeldung: **11.03.2015**
(21) Anmeldenummer: 14183665.0
(22) Anmeldetag: 05.09.2014
(51) Int. Cl.: A61K 8/24, A61K 6/033, A61K 8/02

(54) **Formulierungen und Kit zur biomimetischen Abscheidung von Apatit auf Zähnen**

(30) Priorität: 09.09.2013 DE 102013109846
(71) Anmelder: Heraeus Kulzer GmbH, 63450 Hanau (DE)
(72) Erfinder: Busch, Susanne, 61267 Neu Anspach (DE); Utterodt, Andreas, 61267 Neu Anspach (DE); Gerlach, Michael, 65719 Hofheim (DE)
(74) Vertreter: Bendele, Tanja

(57) **Zusammenfassung**

Es werden Formulierungen zur biomimetischen Abscheidung von Apatit aus einem teilelastischen Formkörper auf Zähnen vorgeschlagen, wobei der Formkörper a) mindestens eine Mineralisationsmatrix enthaltend ein Gel umfasst, das wasserlösliche Phosphate oder zu wasserlöslichen Phosphat-Ionen hydrolysierbare Phosphate und einen pH-Wert von 2 bis 8 aufweist, optional Fluoride und b) die mindestens eine oder eine zweite Mineralisationsmatrix ein zweites Gel mit einem pH-Wert von 3.5 bis 14 aufweist, umfassend Calcium-Ionen oder Calcium-Ionen freisetzende Verbindungen. Darüber hinaus wird das Verfahren zur Herstellung der Formulierung zur Abscheidung von Apatit beansprucht, insbesondere von nadelförmigen Fluorapatitkristalliten. Mit den erfindungsgemässen Formulierungen ist es möglich innerhalb einer Schlafperiode grösser gleich 1 µm Apatit, insbesondere Fluorapatit auf Zahnoberflächen abzuscheiden, um poröse Zahnoberflächen zu verschliessen oder aufzuhellen.

## Beschreibung

Es werden Formulierungen zur biomimetischen Abscheidung von Apatit aus einem teilelastischen Formkörper auf Zähnen vorgeschlagen, wobei der Formkörper a) mindestens eine Mineralisationsmatrix enthaltend ein Gel umfasst, das wasserlösliche Phosphate oder zu wasserlöslichen Phosphat-Ionen hydrolysierbare Phosphate und einen pH-Wert von 2 bis 8 aufweist, optional Fluoride und b) die mindestens eine oder eine zweite Mineralisationsmatrix ein zweites Gel mit einem pH-Wert von 3.5 bis 14 aufweist, umfassend Calcium-Ionen oder Calcium-Ionen freisetzende Verbindungen. Darüber hinaus wird das Verfahren zur Herstellung der Formulierung beansprucht. Mit den erfindungsgemässen Formulierungen ist es möglich innerhalb einer Schlafperiode grösser gleich 1 µm Apatit, insbesondere Fluorapatit auf Zahnoberflächen abzuscheiden, um poröse Zahnoberflächen zu verschliessen oder aufzuhellen.

Zähne sind harte Biomaterialien in Form von Verbundstoffen basierend auf Proteinen und Apatit umfassend Calcium und Phosphat. Der Zahnschmelz, die äussere_Schicht der Zahnkrone ist der härteste Teil des Zahns und enthält keine lebenden Zellen. Er besteht aus anorganischen Kristallen, welche typische hoch orientierte Anordnungen aufweisen. Zahnschmelz ist ein Gewebe, das, sobald es einmal gebildet ist, lebenslang nahezu unverändert bleibt, da die Zellen, welche beim Aufbau der Zähne beteiligt sind, absterben, sobald die Zahnbildung abgeschlossen ist. Ein fertiger Zahnschmelz besteht aus etwa 95 Gew.-% Apatit, etwa 3 Gew. -% Proteinen und Lipiden und etwa 2 Gew. -% Wasser.

Um Schädigungen von Zähnen, insbesondere durch Karies, zu vermeiden oder zu reparieren, wurde seit langem versucht, remineralisierende Systeme einzusetzen. Dabei wurde zunächst versucht, durch Aufbringen von Calciumphosphat-verbindungen die Beschaffenheit der Zähne zu verbessern.

Solche Einkomponentensysteme, bei denen versucht wird, bereits vorgefertigtes Zahnmaterial, beispielsweise Apatit, Hydroxyapatit oder andere Calciumphosphat-verbindungen auf die Zähne aufzubringen, sind unter anderem in EP 0 666 730 B1 oder WO 01/95863 beschrieben. Das Problem solcher Systeme besteht darin, dass das Behandeln von Zahnmaterial mit Calciumphosphat-Verbindungen nicht zu einem Aufwachsen von strukturell dem Zahnmaterial ähnlichen Apatit führt, sondern vielmehr höchstens zu einer blossen Anlagerung von Apatitkristallen auf dem Zahnmaterial, wobei die Apatitkristalle eine vom Zahnmaterial völlig unterschiedliche Morphologie aufweisen. Somit wird keine Festigung des Zahnschmelzes oder dauerhafte Füllung von Läsionen bewirkt, da die angelagerten Apatitkristalle keine ausreichende Ähnlichkeit und Haftung zum natürlichen Zahnmaterial aufweisen.

Bedingt durch moderne Ernährungsgewohnheiten die häufig saure Lebensmittel enthalten, nehmen nicht bakteriell bedingte Erosionen an der Zahnhartsubstanz zu [Dentale Erosionen: Von der Diagnose zur Therapie, Adrian Lussi, Thomas Jaeggi, Quitessenz Verlag].

Nicht nur Lebensmittel wie stark gesäuerte Süssigkeiten, Softdrinks oder Alkopops spielen hier eine Rolle, sondern auch eine zunehmend stark obsthaltige Ernährung kann zu Zahnproblemen führen. Durch die kontinuierliche Einwirkung von Säuren wird der Zahnschmelz dünner und auch poröser. In Extremfällen kann der Schmelz vollständig aufgelöst bzw. abradiert werden, das empfindliche Dentin liegt dann frei. Insbesondere im Zahnhalsbereich, der nur von einer sehr dünnen Schicht Schmelz geschützt wird, tritt dies häufig auf. Die säurebedingte Erosion kann dann noch schneller weiter voranschreiten, da Dentin säurelöslicher ist als Schmelz und es kommt häufig zu keilförmigen Defekten der Zahnhartsubstanz. Freiliegendes Dentin führt zu sensiblen, schmerzempfindlichen Zähnen. Sensible Zahnhälse können aber auch die Folge falscher Putzgewohnheiten sein. Auch zunehmendes Alter ist ein Grund für dünner werdenden Schmelz. Regelmässiges Knirschen kann den Schmelz an den Schneidekanten abradieren. Da durch verbesserte Prophylaxe in der Zahnheilkunde und die konsequente Fluoridierung der meisten Zahncremes und zusätzlicher Pflegeprodukte die Karies zurück geht, die Bevölkerung in den Industriestaaten aber immer älter wird und die funktionsfähigen Zähne länger arbeiten müssen, wächst auch die Bedeutung von nicht kariogenen Zahnhartsubstanzverlusten.

Einige Darreichungsformen, die als geeignet beschrieben werden, die Mineralisation von Apatit auf der Zahnoberfläche anzuregen, sind bekannt. U.S. Pat. No. 6,521,251 beschreibt eine Zusammensetzung, die neben Carbamidperoxid auch Calziumphosphate enthält, deren Löslichkeit etwas besser ist, als die von Apatit, wie Mono-Di-oder Trikalziumphosphat. Dennoch sind all diese Calziumphosphate nur wenig wasserlöslich, daher ist insbesondere bei den beschriebenen Zahnputzmitteln eher eine abrasive als eine remineralisierende Wirkung zu erwarten. Tatsächlich beschreibt U.S. Pat. 5,851,514 unter anderem die Zugabe von Dikalziumphosphat als Abrasivum.

U.S. Pat. No. 6,419,905 erwähnt die Zugabe von Kaliumsalzen (z. B. Citrat) und Fluorid zum Peroxid. Fluorid ist geeignet, um aus dem Speichel insbesondere Kalzium- und Phosphat-Ionen zu binden, wodurch Fluorapatit ausfällt. Wenn keine weiteren Ionen zugefügt werden, wird auch die Bildung von CaF₂ beobachtet. Die Calziumfluoridpartikel können in der Plaque gespeichert werden und über längere Zeit Fluorid abgeben, da sie löslicher sind, als der Apatit der Zahnhartsubstanz. Jedoch werden Zähne durch die gewissenhafte wiederholte tägliche Reinigung von Plaque weitgehend befreit. Damit ist die Wirkung von Calciumfluorid nur von kurzer Dauer und die fluoridhaltigen Produkte müssen regelmässig angewendet werden. Eine Bildung von neuem Apatit wurde mit solchen Produkten nicht beobachtet.

Patent JP20000051804 beschreibt den parallelen Einsatz von konzentrierter Phosphorsäure, konz. H₂O₂ und Fluorapatitpulver. Problematisch erscheint dabei der Einsatz der konzentrierten Phosphorsäure, die gesunden Zahnschmelz merklich auflösen kann. Zudem ist die Bleichlösung stark reizend und darf nicht in Kontakt mit dem Zahnfleisch kommen, was aber in abgeschwächter Form für alle oxidativ wirkenden Zahnbleichmittel gilt. Zudem führt die wiederholte Anwendung nicht zum Aufbau einer Mineralisationsschicht.

Eine säurefreie Anwendung wird in U.S. 20050281759 beschrieben. Als wesentlicher Inhaltsstoff wird hier Kalziumperoxophosphat vorgeschlagen. Kerngedanke ist hier, dass eine einzige Substanz aufhellend und remineralisierend wirken soll, da parallel zur Oxidation die Freisetzung von Kalzium- und Phosphat-Ionen ausgelöst wird. Nicht klar ist, ob die Salze in der verhältnismässig kurzen Einwirkungsdauer einen nennenswerten Apatitaufbau leisten können. U.S. Pat. No. 6,303,104 beschreibt ein oxidationsmittelfreies Zwei-Komponentensystem aus löslichen Kalzium- und Phosphatsalzen, dass auch eine aufhellende Wirkung haben soll. Die Aufhellung soll durch die Zugabe von Natriumgluconat hervorgerufen werden, welches färbende Metallionen (z. B. Eisen) aus dem Zahnschmelz komplexiert. Beim Vermischen der Komponenten ist sofort mit einer Ausfällung der schwerlöslichen Kalziumphosphate zu rechnen und es ist nicht zu erkennen, warum es zu ausgeprägter Remineralisation kommen soll, zumal es sich bei dem Produkt um eine Zahncreme handelt, die nur wenige Minuten in Kontakt mit den Zahnflächen verbleibt. U.S. Pat. 6,102,050 beschreibt eine Zahnseide mit Titandioxidpartikeln, der eine aufhellende, remineralisierende und desensibilisierende Wirkung auf die Interdentalflächen zugeschrieben wird. Es sollen Titandioxidmikropartikel der Grösse 0.1 bis 1.5 µm sowohl als mildes Abrasivum wirken, als auch vom Schmelz absorbiert werden, was mit einer aufhellenden Wirkung verbunden sei. Vermutlich können sich die Partikel allenfalls mechanisch in geeignete Hohlräume einlagern, was keine stabile Verankerung verspricht und nur einen temporären Effekt haben kann.

Alle bisher beschriebenen Patente berücksichtigen nicht, dass Biominerale ihre hohe strukturelle Organisation und Stabilität nur erreichen, weil sie sich in Gegenwart von speziellen Biomolekülen bilden, die Mikro- und Makrostrukturierung vorgeben.

WO 2005/027863 beschreibt ein Zahnpflegemittel, dass über reinigende, remineralisierende, desensibilisierende und aufhellende Wirkung verfügen soll. Als aktive Komponente zur Remineralisation und Aufhellung wird ein nanoskaliges Apatit-Gelatine-Komposit vorgeschlagen, das in Gegenwart einer wässrigen Gelatinelösung gefällt wird und daher Polypeptide eingelagert hat. Dieses Material soll durch sogenannte "Neomineralisation" auf der Zahnoberfläche einen Schutzfilm von dentinähnlicher Struktur bilden können, der eine Oberflächenglättung bewirken soll und offene Dentintubuli verschliessen können soll. Diese Wirkung ist für eine Zahncreme nicht nachvollziehbar, da bevorzugt nur 0.01-2 Gew.-% "Nanite" (WO 01/01930) in diesem Zahnpflegemittel enthalten sind. Die Einwirkung der aktiven Substanzen liegt bei nur maximal einigen Minuten täglich. Eine ausgeprägte oder flächendeckende Mineralabscheidung wird bezweifelt. Eine Mineralabscheidung auf Schmelz ist zudem nicht beschrieben. Ein kontinuierlicher Zuwachs der Filmdicke bei längerer Anwendung des Pflegemittels wird ebenfalls nicht erwähnt. Zudem ist die poröse, wenig geordnete Struktur von Dentin nicht in der Lage, den Zahn vor korrosiven Angriffen zu schützen. Das kommerziell erhältliche Produkt Tooth Mousse oder Mi-Paste basiert auf Patentschriften von Reynolds [WO 98/40406] und soll porösen Schmelz remineralisieren. Die Erfindung beruht darauf, dass Casein (CPP) eine stabilisierende Wirkung auf amorphes Calciumphosphat (ACP) besitzt. Der Wirkstoff CPP-ACP soll in Kontakt mit Zahnhartsubstanz zu Hydroxylapatit remineralisieren. Ein solcher Schutzfilm von dentinartiger Struktur erscheint nicht geeignet, um einen dauerhaften Schutz zu bieten.

Allen Patenten ist gemein, dass sie nur von einer Remineralisation sprechen ohne diese zu belegen und/oder desensibilisierende Wirkung haben. US 2012/0027829A1 beschreibt die Bildung von Hydroxylapatitschichten (HAP) auf Dentin, wenn wiederholt pastenförmige Mischungen aus Propylenglykol, Glycerin, Xylitol, Polyethylenglykol, Cethylpyridiniumchlorid, Tetracalciumphosphat und ein Alkalisalz der Phosphorsäure auf die Zähne aufgebracht werden. Da Tetracalciumphosphat in Gegenwart von Wasser sofort mit den Salzen der Phosphorsäure reagiert, werden zuvor zwei getrennte Pasten hergestellt die erst kurz von der Anwendung gemischt werden. Eine Bildung von HAP auf Schmelz wird nicht beschrieben und es gibt keine Daten zur gebildeten Schicht, die bei eigenen Versuchen auch nicht nachstellbar war.

Die Möglichkeit einer, bei wiederholter Anwendung immer dicker werdenden Fluorapatit-Schicht mit Zahnschmelz-ähnlicher Festigkeit bietet die in US2005220724 und DE 10 2004 054 584.7 beschriebene Technik. Wasserlösliche Phospat- und Fluorid-Salze werden in dem gepufferten Gel A, Kalzium-Ionen im Gel B eingearbeitet. Optional durch eine ionenfreie Schutzschicht getrennt, werden die bei physiologischer Temperatur festen Gelatine-Gele unter Erwärmung nacheinander auf Zahnoberflächen aufgetragen. In Abhängigkeit von den Austauschzyklen der Gele kann eine Zunahme der Schichtstärke beobachtet werden. Die Wachtumsgeschwindigkeiten betragen 0.5 bis 5.0 µm/Tag. Die biologischen Strukturen des Zahnmaterials werden individuell vom Fluorapatit abgebildet, Hohlräume durch offenliegende Dentintubuli werden nach einigen Austauschzyklen verschlossen.

Hinsichtlich einer Anwendung am Menschen erweist es sich als ungünstig, dass die Gele vor der Applikation erwärmt werden müssen. Durch das Aufbringen der zweiten und dritten Gelschicht können darunter liegende, bereits applizierte Gelschichten wieder verflüssigt werden und sich in unerwünschter Weise mit darüber liegenden Schichten vermischen. Insbesondere kleinere Darreichungsmengen trocknen bei Exposition an der Luft schnell aus und ein Verflüssigen durch Erwärmung ist dann nicht mehr ohne weiteres möglich. Die Methode erlaubt kaum, genau definierte Gelmengen gleicher Dicke auf den Zahn aufzubringen. Ferner tragen die drei bis zu 6 mm dicken Gelschichten stark auf, was bei Schutzsystemen wie Schienen oder Pflastern zu Problemen führt, da hier Platz für grosse Gelreservoirs geschaffen werden muss.

Die dort offenbarte Methode zunehmend aufwändig, wenn der gesamte Kiefer mit allen Zahnflächen behandelt werden soll. Da zur Bildung von Fluorapatit idealer Weise eine Anwendungsdauer von 8 Stunden nicht unterschritten werden sollte, wäre es von Vorteil, wenn der Patient das System selbst anwenden könnte, indem er es vor dem Schlafengehen anwendet. Dazu müsste er die Gele erwärmen und präzise auf den Zähnen platzieren, was sehr schwer möglich ist, da erwärmte flüssige Gelatine sehr klebrig ist. Zudem ist die Verbrennungsgefahr gross. Ein weiterer Nachteil ist, dass die Gele im Mundmilieu flüssig bleiben und nicht sicher auf den Zähnen haften.

Da die Gele trotz eines Schutzes wie z.B. einer individualisierten Tiefziehschiene auslaufen, muss die Schiene mit einem geeigneten Abdichtsystem abgedichtet werden, was die Methode noch komplizierter macht.

Die Verwendung von vorgefertigten Gelstreifen DE 10 2006 055 223 A1 hat den Vorteil, dass das aufwändige Erwärmen entfällt und die Streifen die gleiche Dicke haben. Ein grosser Nachteil ist allerdings, dass die Streifen nur Teilareale der Zähne erreichen. Da Erosionen aber mehr oder weniger alle Flächen der Zähne betreffen können, wäre es wünschenswert, dass das Mineralisations-Kit auch überall wirken kann. Ferner ist es sehr umständlich die zwei Streifen auszupacken und beispielsweise in eine Tiefziehschiene einzubringen, die überdies noch ein Reservoir für die Gelstreifen haben muss. Das Problem der Abdichtung der Schienen ist nicht zufriedenstellend gelöst. Da die Streifen sich unter Mundmilieu verflüssigen, ist eine Abdichtung aber notwendig, da die Wirkstoffe sonst in den Mundraum auslaufen können.

Die Erfindung beschreibt eine Methode basierend auf den Patenten US2012195941 (A1) und US2008241797 (A1) mit der eine schmelzähnliche Schicht aus Fluorapatit auf Zähne aufgebracht werden kann. In der beschriebenen Ausführungsform sind zwei Mineralisationsstreifen so geformt, dass einzelne Zahnflächen, eine Gruppe von Zähnen oder mehrere Zahnflächen auf einmal behandelt werden können. Möglich ist auch eine Behandlung von Zahnflächen ganzer Kiefer.

Hintergrund dieser Erfindung war das Ziel Formulierungen für ein Mineralisations-Kit anbieten zu können, der mit hoher Reproduzierbarkeit qualitativ hochwertige zahnschmelzähnliche Überzüge aus Apatit auf Zahnhartsubstanz erzeugt mit dem Ziel diesen vor übermässigem Zahnschmelzverlust zu schützen. Die Inhaltsstoffe sollen weitgehend dem biologischen Vorbild entsprechen und die Anwendung soll möglichst einfach sein. Weitere Aufgaben waren die Anwendung deutlich zu vereinfachen, vorzugsweise soll eine Anwendung sowohl beim Zahnarzt als auch direkt durch den Anwender möglich sein. Zudem soll die Anwendungsdauer der einzelnen Anwendung eines Kits erhöht werden. Daher bestand eine Aufgabe, Formulierungen zu entwickeln, die weitgehend Biomoleküle oder Verbindungen biologischen Ursprungs umfassen, für eine längere Anwendung besonders verträglich sind und zu keinen Irritationen der Mundschleimhaut führen. Ferner sollte die Zeit für eine vorteilhaft flächendeckende Abscheidung vermindert werden, d.h. die Wachstumsgeschwindigkeit des Apatits positiv beeinflusst werden. Auch sollte vorzugsweise die Problematik der Abdichtung der Gele gelöst werden und eine Formulierung bereitgestellt werden, in der vorzugsweise auf eine separate Abdichtung der Zahnschiene verzichtet werden kann, ohne die Abscheidung des Apatits zu verhindern.

Gelöst werden die Aufgaben durch eine Formulierung nach Anspruch 1 und ein Kit nach Anspruch 19 sowie durch die Verfahren zur Herstellung der Formulierung nach den Ansprüchen 14 und 15.

Die Aufgaben wurden gelöst, indem die auf Basis von denaturiertem Kollagen oder anderen Gelbildnern und Mineralstoffen mindestens eine zusammengesetzte Mineralisationsmatrix mit einer 2-Hydroxycarbonsäure mit einem Kohlenwasserstoffrest hergestellt wird. Bevorzugt sind dabei 2-Hydroxycarbonsäuren mit einer guten Löslichkeit in Wasser und insbesondere in den Gelbildner. Vorzugsweise weist die Säure einen pKs-Wert von 3 bis 6 auf. In dem Calciumionen enthaltenden Gel wird vorzugsweise Tris-Puffer eingesetzt und der pH-Wert auf eine Bereich von 4 bis 14, insbesondere 6 bis 11, besonders bevorzugt auf 7,2, bis 9,0 eingestellt. Das Phosphat-Ionen umfassende Gel wurde wird vorteilhaft mit einem Natrium-Acetat Puffer auf einen pH-Wert im Bereich von 2 bis 8, vorzugsweise 2 bis 5,5 oder 3,7 bis 5,7 eingestellt.

Es wurde gefunden, dass bei einer Verwendung von Formulierungen enthaltend Milchsäure, die Apatitschichten besser wachsen als bei einer Verwendung der anderen Carbonsäuren. In Anwendungstests, die auch mit Essigsäure hergestellte Formulierungen umfasste, hat sich gezeigt, dass die Formulierungen der Milchsäure besser verträglich waren. Dies wird zum Teil auch darauf zurückgeführt, dass eine mit Essigsäure oder anderen geruchsintensiven Säuren hergestellten Formulierungen als unangenehmer wahrgenommen werden.

Die Verträglichkeit der Carbonsäuren ist für eine Anwendung wesentlich, da die Formulierungen über einige Stunden im Mund verbleiben und zu keinen Haut- oder Schleimhautirritationen führen sollen. Ein weiterer Vorteil der Milchsäure in den erfindungsgemäßen Formulierungen ist ihre konservierende Wirkung. Um eine konservierende Wirkung zu entfalten ist bei schwachen Säuren deren pH-Wert und ein saurer pH-Wert in der Formulierung notwendig, da nur undissoziierte Moleküle durch die Zellmembran in das Innere von Mikroorganismen eindringen können.

Erfindungsgemäß ist es daher besonders bevorzugt, wenn Milchsäure in der Phosphat-Komponente eingesetzt wird.

Nach einer Alternative ist es bevorzugt die Mineralisationsmatrix durch chemische Modifikation im Mundmilieu unlöslich zu machen, insbesondere als eine Formulierung mit getrennten Formkörpern oder als mehrteiliger Formkörper umfassend jeweils mindestens eine oder zwei Mineralisaitionsmatrices. Überraschend wird die Mineralisationsaktivität trotz der Modifizierung positiv beeinflusst, da das Gel im Mundmilieu nicht mehr verläuft und während der gesamten Behandlungsdauer gleichmäßig wirken kann. Diese chemische Modifikation erfolgt vorzugsweise durch zumindest teilweise oberflächliche chemische Vernetzung der Mineralisationsmatrix, so dass mindestens eine teilweise chemisch vernetzte Ebene oder teilweise Hülle gebildet wird. Erfindungsgemäss wirken die vernetzten Ebenen oder die Hülle wie eine Membran, durch die wässrige Medien, wie Speichel, in die Mineralisationsmatrix eindringen können und zugleich eine Abscheidung von Apatit ausserhalb der Mineralisationsmatrix auf den Zahnoberflächen erfolgen kann.

Die gebildete Ebene oder Hülle kann sich erfindungsgemäss einerseits durch ihre sehr dünne Schicht optimal bei einer Quellung im Mund an die Oberflächen der Zähne anlegen und begünstigt auch die Abscheidung von Apatit im Bereich der Zahnzwischenräume. Ein weiterer besonderer Vorteil der oberflächlichen Vernetzung der Mineralisationsmatrix, bei der sich ein Formkörper ausbildet, ist, dass ein zumindest teilelastischer Formkörper gebildet wird, der sich optimal der Oberflächenkontur von Zähnen und Zahnreihen anschmiegen kann. Besonders gut liegt der teilelastische Formkörper der Mineralisationsmatrix durch die leichtere Verformbarkeit bei Körpertempertur im Mundmilieu an den Zahnoberflächen an. Um den Formkörper, umfassend mindestens eine Mineralisationsmatrix, optimal an den bukkalen, labialen, mesialen und/oder approximalen Oberflächen der Zähne zu fixieren, wird vorteilhaft der mindestens eine Formkörper in eine Zahnschiene eingelegt bzw. in der Zahnschiene bereitgestellt. Durch eine Fixierung mit einer Zahnschiene gelingt es mit dem erfindungsgemässen Formkörper auch, auf mindestens einem Teil oder nahezu vollständig auf den Zähnen sowohl des Oberals auch des Unterkiefers bukkal, mesial, labial, palatinal, distal sowie approximal Apatit abzuscheiden. Durch den erfindungsgemässen mindestens einen Formkörper kann auf einfache Weise erstmalig einfach durch Einsetzen der Formkörper in eine Schiene und Aufsetzen der Schiene auf die Zähne über Nacht bspw. etwa 8 bis 12 Stunden, optional bis 16 oder 24 Stunden, alternativ auch am Tag eine biomimetische Remineralisierung von mindestens teilweise grösser gleich 1 µm erfolgen, bevorzugt grösser gleich 2 µm, bevorzugt grösser gleich 3 µm, besonders bevorzugt ist eine biomimetische Reminerasilierung von mindestens teilweise, vorzugweise im Mittel, von 1 bis 10 µm, 1 bis 5 µm. Besonders bevorzugt erfolgt die Remineralisierung in der Fläche mit einer möglichst homogen Schichtdicke.

Überraschender Weise wurde festgestellt, dass sich das Mineralisationsprodukt nach der chemischen Stabilisierung gleichmässiger und dichter auf der Zahnoberfläche bildet. Die vernetzte Ebene oder Hülle ist so porös, dass trotz Ausbildung der Hülle vergleichbare Apatitschichten abgeschieden werden können, wie die erfindungsgemässen Beispiele 2 bis 4 und 6 gegenüber den Beispielen 1 und 5 im Vergleich aufzeigen. Es wird davon ausgegangen, dass sich durch die chemische Modifizierung ein weniger temperaturempfindliches und vorzugsweise weniger hydrolyseempfindliches Netz aus Polypeptiden um die Mineralisationsmatrix bildet, dessen Poren jedoch noch gross genug sind, um Moleküle und Ionen durchzulassen, welche die stabile, geordnete Apatitschicht an der Zahnoberfläche bilden. Die chemisch vernetzte Schicht oder Ebene wirkt quasi wie eine Ionen und moleküldurchlässige Membran.

Eine ausreichend dicke, homogene und stabile Apatitschicht lässt sich durch das optimale Zusammenspiel der Komponenten: Mineralsalze, Puffer und pH-Wert erzielen. Der pH-Wert kann besonders verträglich durch die erfindungsgemäß werdende Milchsäure eingestellt werden. Ausschlaggebend sind die korrekt gewählten Konzentrationen bei entsprechendem Vernetzungsgrad. Die erfindungsgemässe Formulierung kann zur Abscheidung von Apatit auf Zähnen oder jeglichen anderen biologischen Oberflächen wie auf einer Knochenmatrix eingesetzt werden.

Die einfachere Anwendung der erfindungsgemässen Formulierungen eröffnet die Möglichkeit, dass sowohl Zahnärzte an Patienten als auch Patienten selbst das System anwenden können. Die Abscheidung einer fluoridreichen Calciumphosphatschicht kann Sensibilitäten an den Zähnen vermindern, sie kann Risse und initiale Porösitäten reduzieren und erhöht die Säurestabilität der Zähne. Initiale Zahnhartsubstanzverluste infolge von Säureerosion können gestoppt bzw. teilweise oder vollständig rückgängig gemacht werden. Der erhöhte Fluoridgehalt im Vergleich zu unbehandelten Zähnen in den Schichten reduziert die Löslichkeit des neu gebildeten Minerals. Der natürliche Schmelz wird durch die Schutzschicht vor Zahnbürstenabrasion geschützt.

Gegenstand der Erfindung ist eine Formulierung zur Abscheidung von Apatit, insbesondere geeignet zur biomimetischen Abscheidung von Apatit, ausgewählt aus Fluorapatit (Ca₅[F|(PO₄)₃), Hydroxylapatit (Ca₅[F|(PO₄)₃) oder deren Gemischen auf Zähnen oder auch auf einer Knochenmatrix von Wirbeltieren, wobei die Formulierung mindestens einen teilelastischen Formkörper, insbesondere dreidimensionalen, bevorzugt flächigen Formkörper, aufweist, umfassend mindestens eine Mineralisationsmatrix enthaltend mindestens ein Gel, wobei das Gel mindestens eine Carbonsäure oder eine Mischung von Carbonsäuren ausgewählt aus an C-2 mit einem Kohlenwasserstoff substituierten 2-Hydroxycarbonsäuren aufweist, und
a) die mindestens eine Mineralisationsmatrix ein Gel enthaltend wasserlösliche Phosphate oder zu wasserlöslichen Phosphat-Ionen hydrolysierbare Phosphate und einen pH-Wert von 2 bis 8 aufweist (Phosphat-Komponente), und
b) die mindestens eine oder eine zweite Mineralisationsmatrix ein zweites Gel umfassend Calcium-Ionen, insbesondere wasserlösliche Calcium-Ionen enthaltende Verbindungen, oder Calcium-Ionen freisetzende Verbindungen (Calcium-Komponente) mit einem pH-Wert von 3.5 bis 14 aufweist, umfassend Calcium-Ionen oder Calcium-Ionen freisetzende Verbindungen (Calcium-Komponente), besonders bevorzugt liegt die mindestens eine Mineralisationsmatrix in einem ersten Formkörper I. und die zweite Mineralisationsmatrix in einem zweiten Formkörper II. vor, alternativ liegen zwei Mineralisationsmatrices in einem Formkörper vor.

Vorzugsweise weist der Formkörper zumindest teilweise in mindestens einer Ebene eine gegenüber wässrigen Medien verminderte Löslichkeit im Vergleich zur Mineralisationsmatrix auf.

Carbonsäuren ausgewählt aus an C-2 mit einem Kohlenwasserstoff-Rest substituierten 2-Hydroxycarbonsäuren umfassen vorzugsweise als Kohlenwasserstoff-Rest substituierte oder unsubstituierte Kohlenwasserstoff-Reste, bevorzugt lineare, verzweigte und/oder cyclische Alkyl-, Alkylaryl-, Alkylen-, Alkylenaryl- oder Aryl-Gruppen mit 1 bis 10 C-Atomen, besonders bevorzugt sind unsubstituierte Alkyl-und/oder Aryl-Gruppen. Aufgrund der besseren Löslichkeit sind kurzkettige Alkyl-Gruppen als Kohlenwasserstoffrest an C-2 besonders bevorzugt. Besonders bevorzugt sind Alkyl-Gruppen mit 1 bis 10 C-Atomen, vorzugsweise mit 1, 2, 3, 4, 5 oder 6 C-Atomen.

Besonders bevorzugte Carbonsäuren gemäß der Erfindung weisen einen pKs-Wert von 3 bis 6, insbesondere von 4 bis 5 auf. Konkret bevorzugt Carbonsäuren sind ausgewählt aus (S)-2-Hydroxycarbonsäuren, (R)-2-Hydroxycarbonsäuren, (RS)-2-Hydroxycarbonsäuren oder Gemischen enthaltend diese, insbesondere ist die 2-Hydroxycarbonsäure ausgewählt aus 2-Hydroxypropansäure, (R-)-Milchsäure, (S)-Milchsäure, (RS)-Milchsäure, (S)-2-Hydroxy-3-methyl-butandisäure, (R)-2-Hydroxy-3-methyl-butandisäure, (RS)-2-Hydroxy-3-methyl-butandisäure, (S)-2-Hydroxy-pentan-säure, (R)-2-Hydroxy-pentansäure, (RS)-2-Hydroxy-pentansäure, (S)-2-Hydroxy-pentandisäure, (R)-2-Hydroxy-pentandisäure, (RS)-2-Hydroxy-pentandisäure, (D)-2-Phenyl-2-hydroxyethansäure, (L)-2-Phenyl-2-hydroxyethansäure, (DL)-2-Phenyl-2-hydroxyethansäure (D- und L- Mandelsäure) oder Gemischen enthaltend diese. und Mischungen umfassend mindestens zwei der genannten Säuren oder Phosphatpuffer. Zwekcmäßigerweise könne auch 2-Hydroxy-3-methyl-butandisäure (Citramalsäure), 2-Hydroxy-pentansäure 2-Hydroxy-pentandisäure.

Entsprechend einer bevorzugten Ausführungsform der Erfindung liegen die mindestens eine oder die zwei Mineralisationsmatrix jeweils unabhängig umfassend mindestens ein Gel in Form eines Flächenelements oder mindestens teilweisen Kiefernegativs vor. Ferner ist es bevorzugt, wenn der mindestens eine oder beide teilelastische Formkörper ebenfalls in Form eines Flächenelements oder mindestens teilweisen Kiefernegativs vorliegen.

Bevorzugt ist es, wenn die genannte mindestens eine Ebene eine an der äusseren Oberfläche angeordnete Ebene oder eine im Wesentlichen vollständige äussere Hülle ist.

Erfindungsgemässe Formulierungen umfassen ein oder zwei Formkörper in Form eines Flächenelements mit jeweils einer einschliessenden Hülle mit verminderter Löslichkeit, die insbesondere als Membran fungiert. In einer alternativen Ausführungsform umfasst die Formulierung einen Formkörper in Form eines Flächenelements mit jeweils einer einschliessenden Hülle mit verminderter Löslichkeit, die insbesondere als Membran fungiert, mit zwei aufeinander liegenden Mineralisationsmatrices jeweils umfassend ein Gel, wobei ein Gel die Phosphat- und ein Gel die Calcium-Komponenten umfasst. Diese beiden Mineralisationsmatrices werden von den Formkörpern A und B des Kits umfasst. Entsprechend einer weiteren alternativen Ausführungsform umfasst ein Flächenelement eine Mineralisationsmatrix mit einem Gel, wobei das Gel optional durch eine Membranschicht in zwei Reservoirs unterteilt ist umfassend einmal die Phosphatund einmal die Calcium-Komponente. Die vorgenannten Formkörper liegen vorzugsweise als Flächenelement mit einer Schichtdicke von 5 bis 6000 µm vor, besonders bevorzugt sind Mineralisationsmatrices in Form eines Flächenelements mit jeweils einer Schichtdicke von 5 bis 3000 µm, bevorzugt 100 bis 600 µm, insbesondere 300 bis 600 µm oder um 500 µm, plus/minus 200 µm, insbesondere plus/minus 200 µm, vorteilhaftplus/minus 100 µm oder besser. Die erfindungsgemässen Formkörper umfassen zumindest teilweise eine Hülle oder mindestens eine Ebene mit verminderter Löslichkeit. Statt einem Flächenelement kann der Formkörper auch in Form eines zumindest teilweisen Kiefernegativs vorliegen, vorzugsweise als eine Negativform der Zähne des Ober- und/oder Unterkiefers. Flächenelement der Calcium-Komponente liegen vorzugswiese mit einer Schichtdicke von 500 bis 1500 µm vor, besonders bevorzugt sind Mineralisationsmatrices in Form eines Flächenelements mit jeweils einer Schichtdicke von 500 bis 1200 µm oder um 1000 µm plus/minus 100 µm, insbesondere plus/minus 50 µm. Flächenelement Phosphat-Komponente liegen vorzugsweise mit einer Schichtdicke von 100 bis 1000 µm vor, besonders bevorzugt sind Mineralisationsmatrices in Form eines Flächenelements mit jeweils einer Schichtdicke von 150 bis 800 µm, bevorzugt 300 bis 800 µm, insbesondere 400 bis 600 µm oder um 500 µm plus/minus 100 µm, insbesondere plus/minus 50 µm.

Erfindungsgemässe Formulierungen weisen nach der Vernetzung und wahlweise nach anschliessender Trocknung einen Wassergehalt von 8 bis 60 Gew.-%, bevorzugt von 30 bis 55 Gew.-% auf. Weiter bevorzugt weisen die Gele der Erfindungsgemässe Formulierungen nach der Vernetzung und wahlweise nach anschliessender Trocknung in der Mineralisationsmatrix der Phosphat-Komponente einen Wassergehalt von 20 bis 40 Gew.-%, bevorzugt von 25 bis 35 Gew.-%, besonders bevorzugt um 30 Gew.-% mit einer Abweichung von plus/minus 5 Gew.-% auf. Entsprechend bevorzugt sind Formulierungen der Calcium-Komponente, die nach der Vernetzung und wahlweise nach anschliessender Trocknung in der Mineralisationsmatrix einen Wassergehalt von 30 bis 60 Gew.-%, bevorzugt von 40 bis 60 Gew.-%, vorzugsweise um 50 Gew.-% mit einer Abweichung von plus/minus 5 Gew.-% aufweisen. Die so hergestellten Formulierungen können anschliessend luftund feuchtigkeitsdicht in Blister, Sachets oder dergleichen eingeschweisst werden.

Die Formkörper können bevorzugt in folgenden Alternativen vorliegen: a) zwei Formkörper, insbesondere mit je einer teilweisen, vorzugsweise im Wesentlichen vollständigen Hülle, besonders bevorzugt mit einer umschliessenden Hülle, vorzugsweise zwei Formkörper mit einer Vernetzung der Ober- und/oder Unterseite der als Flächenelement vorliegenden Formkörper, b) ein Formkörper mit zwei Mineralisationsmatrices, der vorzugsweise durch eine Membran getrennt, c) ein Formkörper mit einer Mineralisationsmatrix umfassend zwei Gelbereiche, die optional durch eine Membran getrennt sind. Durch Verwendung von zwei Mineralisationsmatrices oder zwei Gelbereiche für die Phosphat-Komponente ist die Einstellung eines Konzentrationsprofils im Formkörper möglich. Entsprechend kann auch ein Konzentrationsprofil für einen Formkörper enthaltend die Calcium-Komponente eingestellt werden. Die Hülle oder die Ebene weist eine gegenüber wässrigen Medien verminderte Löslichkeit als die Mineralisationsmatrix auf, vorzugsweise fungiert die Hülle als Membran und vermindert ein Auflösen der Mineralisationsmatrix im Mundmilieu. Jedoch kann H₂O aus dem Speichel eindringen, und Fluoride, Calcium- und Phosphat-Ionen sowie Komposite oder Polypeptide können durch die Hülle diffundieren und auf der Zahnoberfläche als Apatit, Hydroxylapatit oder Fluorapatit abgeschieden werden. Die Hülle umschliesst eine wasserreiche Gelatinematrix aus der die Komposite, ionenbeladene Wassermoleküle bzw. hydratisierte Ionen heraus diffundieren können. Die Diffusion der Komposite ist wichtig, denn die Komposite sind mit organischen Makromolekülen substituierte Hydroxyapatite, die den Zahnschmelz bilden und, um diese auf den Zahnoberflächen abzuscheiden. Erfindungsgemäss werden Fluorapatit-Protein-Komposite aus den Formkörpern auf den Zahnoberflächen abgeschieden.

Der aus der erfindungsgemässen Formulierung abgeschiedene Fluorapatit liegt vorzugsweise auf den Zähnen kristallin vor, bevorzugt microkristallin, besonders bevorzugt in Form von nadeligen Kristalliten. Die innerhalb eines Anwendungszyklus von etwa 8 Stunden auf den Zahnoberflächen abgeschiedene zumindest teilweise Apatitschicht, vorzugsweise durchgängige Apatitschicht, weist mindestens eine Schichtdicke von 1 µm auf. Ebenso im Rahmen der Erfindung sind zumindest teilweise nicht durchgängige Apatitschichten, die unregelmässig bis vorzugsweise homogen zumindest einen Teil der behandelten Zahnoberfläche bedecken. Die Apatitschichten können zumindest teilweise bis zu 15 µm betragen, vorzugsweise werden im Wesentlichen vorzugsweise homogene Apatitschichten von größer gleich 1 µm, bevorzugt größer gleich 2 µm, vorzugsweise größer gleich 5 µm bis 13 µm, und im Mittel von 1 bis 10 µm erhalten.

Unter einer gegenüber wässrigen Medien verminderten Löslichkeit der chemisch vernetzten Ebene oder Hülle im Vergleich zur Mineralisationsmatrix wird folgendes verstanden: Die chemisch unvernetzte Mineralisationsmatrix und optional nur mit einem Weichmacher modifizierte Mineralisationsmatrix, bspw. die mit Glycerin über Wasserstoffbrückenbindungen vorzugsweise als Addukt vernetzte Gelatine. Bevorzugt wird Gelatine der folgenden Qualitäten eingesetzt von Bloom 175 bis 300, oder höher, bevorzugt ist Gelatine Bloom 300 (Schweineschwarte).

Unter Zähnen von Wirbeltieren werden erfasst menschliche Zähne, Prothesen menschlicher Zähne, Milchzähne (*Dentes decidui*), Bleibende-Zähne (*Dentes permanentes*), Kronen, Inlays, Implantate, Zähne von Tieren, wie Haustieren und Nutztieren wie Hunden, Pferden, Katzen.

Unter einem mindestens teilelastischen Formkörper wird im Sinne der Erfindung ein dreidimensionaler Formkörper verstanden, der als Flächenelement oder auch mit jeder beliebigen dreidimensionalen Geometrie vorliegt, insbesondere in Form eines mindestens teilweisen Kiefernegativs, und elastische Eigenschaften aufweist. Als elastisch oder teilelastisch gilt der Formkörper, wenn der Körper unter Krafteinwirkung seine Form verändert und bei Wegfall der einwirkenden Kraft zumindest teilweise oder vollständig wieder in die Ursprungsform zurückkehrt. Die elastische oder teilelastische Eigenschaft weist der Formkörper vorzugsweise bei der Anwendung im Mundmilieu auf und vorzugsweise nach der Herstellung. Bei zu langer Trocknung kann die Elastizität abnehmen.

In einer nach der Erfindung besonders bevorzugten Formulierung weist die Mineralisationsmatrix in a) folgende Zusammensetzung auf: a) die mindestens eine Mineralisationsmatrix weist ein Gel auf, umfassend (i) wasserlösliche Phosphate oder zu wasserlöslichen Phosphat-Ionen hydrolysierbare Phosphate, insbesondere Na₂HPO₄, vorzugsweise liegt der Gehalt an Phosphat in der Mineralisationsmatrix bei 1 bis 10 Gew.-%, vorzugsweise von 2 bis 8 Gew.-%, besonders bevorzugt von 5 bis 8 Gew.-% (ii) einen Gehalt an Wasser oder ein Gemisch von Wasser und einem organischen Lösemittel, (iii) optional mindestens eine 2-Hydroxycarbonsäure mit einem Kohlenwasserstoffrest an C-2, insbesondere eine 2-Hydroxycarbonsäure mit einem Alkylrest an C-2 mit 1 bis 10 C-Atomen, vorzugsweise mit 1, 2, 3, 4 oder 5 C-Atomen, erfindungsgemäß Milchsäure, und optional ein Puffersystem, insbesondere liegt ein Puffersystem zur Einstellung des pH-Wertes im Bereich von 2 bis 8 vor, insbesondere von 3.5 bis 8, bevorzugt von 3.5 bis 6, besonders bevorzugt um 5,5 plus/minus 0,5. Der Gehalt bezieht sich auf PO₄³⁻.

Der Kohlenwasserstoffstamm der 2-Hydroxycarbonsäure kann 2 bis 15 C-Atome umfassen, bevorzugt sind 2 bis 6 C-Atome in der Stammkette, die vorzugsweise eine substituierte Alkyl-Kette ist, wobei Milchsäure als eine 2-Hydroxycarbonsäure mit einem Methyl-Rest an C-2 betrachtet wird, d.h. als eine 2-Methyl-2-Hydroxyethansäure.

Gleichzeitig weist die nach der Erfindung besonders bevorzugte Formulierung eine Mineralisationsmatrix in b) folgender Zusammensetzung auf: die zweite Mineralisationsmatrix oder die mindestens eine Mineralisationsmatrix weist ein zweites Gel auf, umfassend (i) Calcium-Ionen oder Calcium-Ionen freisetzende Verbindungen, insbesondere Calciumdichlorid oder Hydrate davon, vorzugsweise zusätzlich Calciumsulfat, Nanoapatit, Natriumcarbonat oder Calciumoxalat, vorzugsweise liegt der Gehalt an Calcium in der Mineralisationsmatrix bei 1 bis 10 Gew.-%, vorzugsweise größer gleich 1,5 bis 7,5 Gew.-%, (ii) optional Wasser oder ein Gemisch von Wasser und einem organischen Lösemittel, und (iii) optional 2-Hydroxycarbonsäure mit einem Kohlenwasserstoffrest an C-2, insbesondere eine 2-Hydroxycarbonsäure mit einem Alkylrest an C-2 mit 1 bis 10 C-Atomen, vorzugsweise mit 1, 2, 3, 4 oder 5 C-Atomen, erfindungsgemäß Milchsäure, und/oder ein Puffersystem.

Zur Herstellung der Puffer werden bevorzugt Hydroxycarbonsäuren, wie Fruchtsäuren und Alkali-Salze eingesetzt. Der Gehalt bezieht sich auf das Calcium (Ca²⁺).

Weiter ist es bevorzugt, wenn die Formulierung in der mindestens einen Mineralisationsmatrix ein Gel aufweist, umfassend mindestens ein wasserlösliches Fluorid (F⁻), mit Fluorid-Ionen, oder eine Fluoride freisetzende Verbindung. Besonders bevorzugt weist die Formulierung in a) als weitere Komponente (iv) mindestens ein wasserlösliches Fluorid oder eine Fluoride freisetzende Verbindung auf.

Nach einer bevorzugten Ausgestaltung der Erfindung umfasst das mindestens eine wasserlösliche Fluorid oder die mindestens eine Fluoride freisetzende Verbindung, insbesondere in a) die mindestens eine Mineralisationsmatrix, (i) mindestens eine unsubstituierte oder substituierte Alkyl-Gruppe aufweisende quaternäre mono- oder poly-Ammonium-Verbindung, vorzugsweise mit vier substituierten Alkyl-Gruppen, wobei die mindestens eine substituierte Alkyl-Gruppe umfasst Hydroxyalkyl-, Carboxyalkyl-, Aminoalkyl-Gruppen mit 1 bis 25 C-Atomen oder organofunktionelle durch Heteroatome unterbrochene Gruppen mit bis zu 50 C-Atomen. Bevorzugte Ammonium-Verbindungen können 1 bis 20 quaternäre Ammonium-Funktionalitäten enthalten, vorzugsweise 1, 2, 3, 4, 5, 6, 7, 8 Ammonium-Funktionalitäten, bevorzugt wird Olaflur (N,N,N'-Tris(2-hydroxyethyl)-N'-octadecyl-1,3-diaminopropandihydro-fluorid) als wasserlösliches Fluorid eingesetzt. Gleichfalls bevorzugt sind Aminfluoride, wie Oleaflur, Decaflur, Ethanolamin-Hydrofluorid, (ii) eine Fluoride freisetzende organofunktionelle Amino-Verbindung oder ein Fluoride freisetzendes Antiseptikum auf Basis von organofunktionellen Amino-Verbindungen, wie insbesondere Fluoride von *N*-Octyl-1-[10-(4-octyliminopyridin-1-yl)decyl]pyridin-4-imin, Cetylpyridinium fluorid, oder c) wasserlösliche anorganische Fluoride, wie Alkalifluoride, Natriumfluorid, Kaliumfluorid, Zinnfluorid, Ammoniumfluorid, oder Fluoride freisetzende anorganische Fluoride, wie Zinkfluorid, Zinkhydroxyfluorid.

Als erfindungsgemässes Gel kann vorzugsweise mindestens ein Gelbilder, ausgewählt aus denaturiertem Kollagen, Hydrokolloiden, Polypeptiden, Proteinhydrolysaten, synthetische Polyaminosäuren, Polysacchariden, Polyacrylaten (Superabsorber) oder Mischungen umfassend mindestens zwei der genannten Gelbilder in der Formulierung vorliegen.

In einer erfindungsgemässen Formulierung umfasst die Mineralisationsmatrix als Gel Gelatine und vorzugsweise ein Weichmacher, bevorzugt ein Polyol, wie Glycerin und/oder deren Umsetzungsprodukte optional in Gegenwart von Wasser. Alternativ können auch Gelatine und ein Weichmacher wie Sorbitol eingesetzt werden. Der Weichmacher bewirkt eine Erhöhung des Schmelzbereiches durch Ausbildung von intermolekularen Wasserstoffbrückenbindungen. Als Gel wird erfindungsgemäss Gelatine vorzugsweise (denaturiertes Kollagen, tierisches Eiweiss, Protein), besonders bevorzugt wird eine sauer hydrolysiertes Kollagen, oder Gelatine und ein Polyol, wie Glycerin, eingesetzt.

Für die Anwendung an Zähnen ist es bevorzugt, wenn die Mineralisationsmatrix und der Formkörper als ein Flächenelement oder mindestens teilweisen Kiefernegativs vorliegen. Ein erfindungsgemässes Flächenelement oder der Formkörper in Form mindestens eines teilweisen Kiefernegativs kann auch eine Oberflächentextur, die den Zahnoberflächen eines Zahnbogens nachempfunden ist, nachbilden.

Verbindungen zur thermischen Stabilisierung des Gels umfassen vorzugsweise Weichmacher basierend auf Polyolen.

Als Verbindungen zur chemischen Vernetzung, insbesondere kovalenten Vernetzung, in der mindestens einen Ebene oder zur Ausbildung der Hülle der Mineralisationsmatrix werden die di- oder polyfunktionellen Vernetzer eingesetzt, bevorzugt Glutardialdehyd. Die chemischen Vernetzer bilden mit den Gelen, insbesondere den Polypeptiden oder Polyaminosäuren kovalente Vernetzungstellen aus. Vorzugsweise umfassen die di- oder polyfunktionellen Vernetzer Dialdehyde, Polyepoxide, und/oder Polyisocyanate sowie Mischungen umfassend mindestens zwei Vernetzer. Weiter bevorzugt werden pharmakologisch unbedenkliche Vernetzer eingesetzt. Bevorzugte Dialdehyde umfassen alpha, omega-Dialdehyde von Kohlenwasserstoffen, insbesondere umfassend 2 bis 50 C-Atome, insbesondere 4 bis 10 C-Atome in der bifunktionellen Alkylen Gruppe. Durch eine Behandlung der Mineralisationsmatrix mit einem Vernetzer verringert sich die Löslichkeit der Gelatine bis auf ein Niveau, dass sie für etwa 8 Stunden im Mundmilieu nicht verflüssigt. Bevorzugt ist die Behandlung mit Glutardialdehyd für mindestens 5s, je nach Anwendung kann die Vernetzung länger erfolgen und damit stärker sein, bspw., wenn eine Mineralisationsmatrix für 12 bis 16 Stunden im Mundmilieu verbleiben soll. Nach 40 s Spülen, Tauchen in oder Sprühen mit einer 0.5%igen Glutardialdehyllösung reduziert sich die Löslichkeit des Gels so weit, dass es bei Mundmillieu für bis zu 8 Stunden nicht verflüssigt. Die optional verwendbaren Membran(schichten) sind ionenfrei.

Die Vernetzungslösung weist vorzugsweise einen Gehalt von etwa 0,25 bis 0,5 Gew.-% Vernetzer, vorzugsweise Glutardialdehyd auf. Die besten Ergebnisse hinsichtlich einer ausreichenden Vernetzung und optimalen Durchlässigkeit für die abzuscheidenden Apatitkomposite hat sich eine Behandlungszeit von, 0 bis 60 s, bevorzugt von etwa 5 bis 40 s, besonders bevorzugt von 10 bis 30 s, erfindungsgemäß um 20 Sekunden (s) gezeigt.

Entsprechend einer bevorzugten Ausführungsform umfasst die Formulierung (I) einen Formkörper in Form eines Flächenelements mit mindestens zwei optional durch eine Membran(schicht) getrennte Mineralisationsmatrices jeweils in Form eines Flächenelements enthaltend das Gel mit dem folgenden Schichtaufbau im Formkörper:
A) eine erste Mineralisationsmatrix in Form eines Flächenelements umfassend Gel und Phosphate oder zu wasserlöslichen Phosphat-Ionen hydrolysierbare Phosphate, wasserlösliche Fluoride oder Fluoride freisetzende Verbindung, Wasser oder ein Gemisch von Wasser und einem organischen Lösemittel, optional mindestens eine 2-Hydroxycarbonsäure mit einem Kohlenwasserstoffrest an C-2, insbesondere eine 2-Hydroxycarbonsäure mit einem Alkylrest an C-2 mit 1 bis 10 C-Atomen, vorzugsweise mit 1, 2, 3, 4 oder 5 C-Atomen, erfindungsgemäß Milchsäure, und optional ein Puffersystem,
B) optional Membran(schicht),
C) eine zweite Mineralisationsmatrix in Form eines Flächenelements umfassend Gel und Calcium-Ionen oder Calcium-Ionen freisetzende Verbindungen, optional Wasser oder ein Gemisch von Wasser und einem organischen Lösemittel, optional mindestens eine Carbonsäure und/oder ein Puffersystem.

Gleichfalls Gegenstand der Erfindung sind Formulierungen umfassend (I) einen Formkörper in Form eines Flächenelements mit mindestens zwei, optional durch eine Membran getrennte Mineralisationsmatrices jeweils unabhängig in Form eines Flächenelements enthaltend das Gel mit einer Schichtdicke von 50 bis 6000 µm, insbesondere von 500 bis 2000 µm, bevorzugt von 1000 bis 2000 µm oder 500 bis 1500 µm, oder (II) zwei separate Formkörper jeweils unabhängig in Form eines Flächenelements mit mindestens jeweils einer Mineralisationsmatrix in Form eines Flächenelements enthaltend das Gel, wobei jeder Formkörper jeweils unabhängig eine Schichtdicke von 10 bis 3000 µm aufweist, vorzugsweise 50 bis 1000 µm, besonders bevorzugt 100 bis 750 µm, vorzugsweise 300 bis 600 µm, besonders bevorzugt um 500 µm plus/minus 300 µm, wobei der erste Formkörper umfassend Phosphate, wie Hydrogenphosphate, oder zu wasserlöslichen Phosphat-Ionen hydrolysierbare Phosphate eine Schichtdicke von 50 bis 3000 µm, insbesondere 100 bis 3000 µm, bevorzugt von 300 bis 1000 µm, besonders bevorzugt um 500 µm plus/minus 200 µm oder +/- 50 µm, und/oder der zweite Formkörper umfassend Calcium-Ionen oder Calcium-Ionen freisetzende Verbindungen eine Schichtdicke von 10 bis 3000 µm aufweist, insbesondere 100 bis 3000 µm, bevorzugt von 300 bis 1000 µm, bevorzugt von 300 bis 750 µm, vorzugsweise um 500 µm plus/minus 200 µm oder +/- 50 µm. Alternativ sind die vorgenannten Formkörper ein Teil eines zumindest teilweisen Kiefernegativs mit einem entsprechenden inneren, an den Zähnen anzuordnenden mehrschichtigen Aufbau der Formkörper.

Zusätzlich zur Milchsäure sowie dem Calcium-Salz der Milchsäure können Glycolsäure, Asparaginsäure und die Äpfelsäure in Mischungen mit Milchsäure oder in Mischungen mit einem Calcium-Salz der Milchsäure verwendet werden.
Für die Puffersysteme werden vorteilhaft Alkali- und/oder Erdalkali-Salze oder Zink-Salze der nachfolgenden Carbonsäuren eingesetzt ausgewählt aus Fruchtsäuren, wie α-Hydroxycarbonsäuren wie Äpfelsäure, Zitronensäure, Glycolsäure, Milchsäure und Weinsäure; Aminosäuren, Fettsäuren, Hydroxycarbonsäuren, Dicarbonsäuren und Mischungen umfassend mindestens zwei der genannten Säuren und/oder das Puffersystem umfasst Carboxylate von Alkylcarbonsäuren, Fettsäuren, Fruchtsäuren, Fumarate, Aminosäuren, Hydroxycarbonsäuren, Dicarbonsäuren und Mischungen umfassend mindestens zwei der genannten Säuren oder Phosphatpuffer. Für die Puffersysteme werden vorteilhaft Alkali- und/oder Erdalkali-Salze oder Zink-Salze eingesetzt.

Die Puffersysteme umfassen EDTA, TRIS: Tris(hydroxymethyl)-aminomethan für pH 7,2 bis 9,0, HEPES: 4-(2-Hydroxyethyl)-1-piperazinethanesulfonsäure für pH 6,8 bis 8,2, HEPPS: 4-(2-Hydroxyethyl)-piperazin-1-propansulfonsäure für pH 7,3 bis 8,7, Barbital-Acetat Puffer, MES: 2-(N-Morpholino)ethansulfonsäure für pH 5,2 bis 6,7, Kohlensäure-Bicarbonat-System für pH 6,2 bis 8,6; neutral, Kohlensäure-Silicat-Puffer für pH 5,0 bis 6,2; schwach sauer, Essigsäure-Acetat-Puffer für pH 3,7 bis 5,7, Phosphatpuffer: NaH₂PO₄ + Na₂HPO₄ für pH 5,4 bis 8,0, Ammoniakpuffer NH₃ + H₂O + NH₄Cl für pH 8,2 bis 10,2, Zitronensäure- oder Zitratpuffer. Besonders bevorzugte Puffersysteme umfassend Milchsäure Puffersysteme, EDTA, oder Barbital-Acetat Puffer und in der Mundspüllösung TRIS (Tris(hydroxymethyl)-aminomethan) Puffer. In der Mundspüllösung synonym zu Vorbehandlungslösung wird TRIS (Tris(hydroxymethyl)-aminomethan) eingesetzt.

Erfindungsgemäss einsetzbare Phosphate zur Herstellung der Phosphat enthaltenden Mineralisationsmatrices umfassen die Phosphate, Hydrogenphosphate, oder zu wasserlöslichen Phosphat-Ionen hydrolysierbare Phosphate umfassend a) Alkaliphosphate, Erdalkaliphosphate, Dihydrogenphosphate, Natriumdihydrogenphosphat, NaH₂PO₄, Kaliumdihydrogenphosphat, KH₂PO₄, Hydrogenphosphate, Dikaliumhydrogenphosphat, K₂HPO, Dinatriumhydrogenphosphat, Na₂HPO₄, Phosphatester, Monoester, Diester und Triester von Phosphaten, Natriumphosphat, Na₃PO₄, Kaliumphosphat, K₃PO₄, Calciumdihydrogenphosphat, Ca(H₂PO₄)₂, Monoester, Diester und Triester Calciumhydrogenphosphat, CaHPO₄, Calciumphosphat, Ca₃(PO₄)₂ und/oder
b) die Calcium-Ionen oder Calcium-Ionen freisetzende Verbindungen umfasst Calciumchlorid, Calciumdichlorid-Dihydrat, Calcium-Salz einer Carbonsäure umfassend Alkylcarbonsäuren, Hydroxycarbonsäure, Dicarbonsäuren, Fruchtsäuren, Aminosäuren, wie Calciumlactat, Calciumgluconat, Calciumlacto-Gluconat, Calciumalginat, Calcium-L-Ascorbat, in Wasser schlecht lösliche Calcium-Ionen retardiert freisetzende Verbindungen umfassend Calciumsulfat, Calciumapatit, Calciumcarbonat, Calciumoxalat, Calciumphosphat, Calciumalginat, vorzugsweise mit einer Partikelgrösse von kleiner 100 µm, bevorzugt um 10 µm, besonders bevorzugt kleiner gleich 5 µm bspw. bis 1 µm oder 50 nm oder vorzugsweise Gemische von wasserlöslichen und schlecht in Wasser löslichen Calcium-Ionen oder Calcium-Ionen freisetzenden Verbindungen. Die in Wasser schlecht löslichen Calcium-Ionen retardiert freisetzenden Verbindungen werden zur Texturverbesserung der teilweise klebrigen Gele dem gut wasserlösliche Calcium-Ionen enthaltendem Gel zugesetzt. In Bezug auf die Gesamtzusammensetzung der Mineralisationsmatrix umfassend das Gel können 1 bis 50 Gew.-% Wasser schlecht lösliche Calcium-freisetzende Verbindungen eingesetzt werden, bevorzugt werden 5 bis 30 Gew.-% eingesetzt.

Gleichfalls Gegenstand der Erfindung ist ein Verfahren zur Herstellung einer Phosphat-Ionen enthaltenden Formulierung sowie eine Formulierung erhältlich nach dem Verfahren zur biomimetischen Abscheidung von Apatit ausgewählt aus Fluorapatit, Hydroxylapatit oder deren Gemischen auf Zähnen von Wirbeltieren, indem,
(1) mindestens ein teilelastischer Formkörper, insbesondere Formkörper A, umfassend mindestens eine Mineralisationsmatrix enthaltend mindestens ein Gel mit wasserlöslichen Phosphaten oder zu wasserlöslichen Phosphat-Ionen hydrolysierbare Phosphate hergestellt wird, und der Formkörper hergestellt wird, indem
   a) zur Herstellung mindestens einer Mineralisationsmatrix enthaltend das Gel, auch Phosphatkomponente A genannt, in einem ersten Schritt eine Mischung von
      (i) 0.05 bis 4 mol/l, 0.5 bis 1.5 mol/l wasserlöslichen Phosphaten oder zu wasserlöslichen Phosphat-Ionen hydrolysierbaren Phosphaten,
      (ii) eine entsprechende Menge Wasser oder ein Gemisch von Wasser und einem organischen Lösemittel,
      (iii) optional mindestens eine 2-Hydroxycarbonsäure mit einem Kohlenwasserstoff-Rest an C-2 und optional ein Puffersystem, insbesondere zur Einstellung des pH-Wertes von 2 bis 8, vorzugsweise 3.5 bis 8, bevorzugt 3.5 bis 6, besonders bevorzugt um 5,5 plus/minus 0,5
      (iv) 0 bis 6000 Gew.-ppm wasserlösliches Fluorid oder eine Fluoride freisetzende Verbindung, insbesondere 1 bis 4000 Gew.-ppm, weiter bevorzugt 500 bis 2500 Gew.-ppm, besonders bevorzugt um 2000 Gew.-ppm plus/minus 500 Gew.-ppm, gemischt werden, in einem weiteren Schritt wird aus der in a) hergestellten Mischung
   b) mit Gelatine und optional Glycerin unter Erwärmen das Gel hergestellt.
   c) Formung unter Ausbildung der Mineralisationsmatrix, optional Verfestigung.

In beiden Verfahren kann in einem nachfolgenden Schritt d) eine Ausbildung einer an der äusseren Oberfläche angeordneten Ebene, insbesondere der Hülle, der mindestens einen Mineralisationsmatrix unter Bildung des Formkörpers erfolgen.

Der pH-Wert der Phosphat-Lösung liegt zwischen 2.0 und 8.0, bevorzugt zwischen 3.5 und 5.5 wird mit einem geeigneten Puffersystem eingestellt. Besonders geeignet sind Carbonsäuren, wie Milchsäure aber auch alle anderen Puffersysteme. Die Konzentration des Puffers liegt zwischen 0.25 und 4.0 mol/l, bevorzugt zwischen 0.5 und 1.5 mol/l.

Aus der Lösung wird ein Gelatine-Glycerin-Gel hergestellt. Die Menge an Gelatine beträgt vorzugsweise 25 bis 40 Gew.-% und die Menge an Glycerin 5 bis 20 Gew.-% in Bezug auf die Gesamtzusammensetzung an wässrigem Gel. Um die Komponenten homogen zu mischen, wird die Zubereitung auf 40 bis 90°C erwärmt bevorzugt auf 50 bis 70°C. Die Dicke der Phosphat-Komponente A beträgt hierbei 50 bis 3000 µm, bevorzugt 200 bis 2000 µm, besonders bevorzugt 300 bis 1500 µm.

Ebenso Gegenstand der Erfindung ist ein Verfahren zur Herstellung einer Calcium-Ionen enthaltenden Formulierung sowie eine Formulierung erhältlich nach dem Verfahren zur Abscheidung von Apatit, insbesondere zur biomimetischen Abscheidung von Apatit, ausgewählt aus Fluorapatit Hydroxylapatit oder deren Gemischen auf Zähnen von Wirbeltieren indem,
(1) mindestens ein teilelastischer Formkörper, insbesondere Formkörper B, umfassend mindestens eine Mineralisationsmatrix enthaltend mindestens ein Gel mit Calcium-Ionen oder Calcium-Ionen freisetzende Verbindungen hergestellt wird und der Formkörper hergestellt wird, indem
   a) zur Herstellung mindestens einer Mineralisationsmatrix enthaltend das Gel, auch Calciumkomponente B genannt, in einem ersten Schritt eine Mischung von
      (i) 0.1 bis 2 mol/l Calcium-Ionen oder Calcium-Ionen freisetzende Verbindungen,
      (ii) eine entsprechende Menge Wasser oder ein Gemisch von Wasser und einem organischen Lösemittel,
      (iii) optional mindestens eine 2-Hydroxycarbonsäure mit einem Kohlenwasserstoff-Rest an C-2 und optional ein Puffersystem, insbesondere zur Einstellung des pH-Wertes von 3.5 bis 14, bevorzugt von 4.0 bis 6.0 oder 6.0 bis 11.0, vorzugsweise 4.0, besonders bevorzugt um 4.0 plus/minus 0,5,
      gemischt werden, in einem weiteren Schritt wird aus der in a) hergestellten Mischung
   b) mit Gelatine und optional Glycerin unter Erwärmen das Gel hergestellt,
   c) Formung unter Ausbildung der Mineralisationsmatrix, optional Verfestigung, und in einem nachfolgenden Schritt d) erfolgt eine Ausbildung einer an der äusseren Oberfläche angeordneten Ebene, insbesondere der Hülle, der mindestens einen Mineralisationsmatrix unter Bildung des Formkörpers. Die Ebene oder Hülle kann durch die vorgenannte Vernetzung oder Auftragen eines Coatings erzeugt werden. Entscheidend bei der Herstellung der Ebene oder der Hülle ist eine gewisse Porosität, um eine Abscheidung der Biokomposite zu ermöglichen.

Zur Herstellung der vorgenannten Formulierungen werden in b) jeweils unabhängig im weiteren Schritt 5 bis 50 Gew.-% Gelatine in Bezug auf die Gesamtzusammensetzung Gel zugesetzt und 0 bis 30 Gew.-% Glycerin in Bezug auf die Gesamtzusammensetzung Gel zugesetzt, bevorzugt werden 25 bis 40 Gew.-% Gelatine und 5 bis 20 Gew.-% Glycerin zur Herstellung der Formulierung enthaltend wasserlösliche Phosphate oder zu wasserlöslichen Phosphat-Ionen hydrolysierbaren Phosphate enthaltenden Matrix, und 20 bis 40 Gew.-% Gelatine und 15 bis 25 Gew.-% Glycerin zur Herstellung der Formulierung enthaltend Calcium-Ionen oder Calcium-Ionen freisetzende Verbindungen enthaltenden Mineralisationsmatrix zugesetzt.

Die Gelatine enthaltenden Formulierungen in Schritt b) werden vorzugsweise auf 40 bis 90 °C erwärmt, um die Komponenten homogen zu vermischen, bevorzugt ist der Temperaturbereich von 50 bis 70 °C.

Die Lösung zur Herstellung der Calciumkomponente B enthält ein wasserlösliches Calciumsalz, z.B. Calciumchlorid oder Calciumlactat oder Calciumgluconat oder Calciumlacto-Gluconat. Die Aufzählung ist einschliesslich aber nicht ausschliesslich. Die Konzentration liegt zwischen 0.1 und 2.0 mol/l, bevorzugt zwischen 0.5 und 1.5 mol/l. Der pH-Wert zwischen 4.0 und 14.0, bevorzugt zwischen 6.0 und 11.0 wird mit einem geeigneten Puffersystem eingestellt. Besonders geeignet sind Carbonsäuren wie Ascorbinsäure, Brenztraubensäure, Weinsäure, Essigsäure, Milchsäure oder Äpfelsäure aber auch alle anderen Puffersysteme mit geeignetem pks-Wert. Die Konzentration des Puffers liegt zwischen 0.1 und 3.0 mol/l, bevorzugt zwischen 0.25 und 1.0 mol/l. Aus der Lösung wird ein Gelatine-Glycerin-Gel hergestellt. Die Menge an Gelatine beträgt vorzugsweise 20 bis 40 Gew-% in Bezug auf die Gesamtzusammensetzung an wässrigem Gel und die Menge an Glycerin 15 bis 25 Gew-%. Da die Calcium-Gelatine-Lösung auch nach Gelieren sehr klebrig ist und damit unangenehm im Handling wird zur Texturverbesserung ein schlecht lösliches Calciumsalz zugegeben. Besonders geeignet sind Calciumsulfat, Calciumapatit, Calciumcarbonat, Calciumoxalat. Die Aufzählung ist einschliesslich aber nicht ausschliesslich. Um eine besonders homogene Paste zu erhalten, ist es vorteilhaft wenn die Partikelgrössen unter 10 µm liegen. Bevorzugt werden Partikel mit Partikelgrössen kleiner 1 µm eingesetzt. Die Zugabe des schwer löslichen Calciumsalzes liegt bevorzugt bei 1 bis 50 %, sehr bevorzugt bei 5 bis 30%. Um die Komponenten homogen zu mischen, wird die Zubereitung unter Rühren auf 40-90°C erwärmt bevorzugt auf 50 bis 70°C.

Die Dicke der Calcium-Komponente A beträgt hierbei 10 bis 3000 µm, bevorzugt 100 bis 1500 µm, besonders bevorzugt 300 bis 1500 µm, vorzugsweise 500 bis 1500 µm.

Zur Herstellung der Formkörper der Formulierungen werden die noch nicht verfestigten Gele geformt und anschliessend verfestigt.

Die Formung der Gele zur Ausbildung der Mineralisationsmatrix kann durch Einfüllen in Formen, Extrusion, Formung zu Flächenelementen durch Aufstreichen, Verteilen, Ausrollen, Pressen durch entsprechend geformte Düsen erfolgen, gefolgt von einem Verfestigungsschritt. Eine Extrusion der Gele in jede beliebige Form ist vorzugsweise mit pastenförmigen Gelen gut möglich. Die Verfestigung erfolgt in der Regel bereits beim Abkühlen.

Der Vernetzung liegt dazu in einer Vernetzerlösung vor und wird mit dem geformten Gel in Kontakt gebracht. Bevorzugt sind Lösungen von 0,005 bis 90 Gew.-% Vernetzer in Lösemittel in Bezug auf die Gesamtzusammensetzung, insbesondere in Wasser oder Wasser enthaltendem Lösemittel, bevorzugt sind 0,005 bis 5 Gew-%, besonders bevorzugt 0,1 bis 4 Gew.-%, vorteilhaft um 0,1 bis 1 Gew.-%. Bevorzugt wird als Vernetzerlösung eine wässrige Glutardialdehyd-Lösung eingesetzt. Die Vernetzerlösung wird vorzugsweise hergestellt, indem die Phosphatlösung mit dem Vernetzer versetzt wird. Die bevorzugte Einwirkzeit liegt bei 1 bis 200 Sekunden, besonders bevorzugt bei 10 bis 60 Sekunden (s). Vorzugsweise wird für etwa 20 Sekunden behandelt. Der bevorzugte pH-Wert der Vernetzungslösung liegt zwischen 4.0 und 12.0. Ein anschliessendes Abspülen mit Phosphat- bzw. Calciumlösung ist möglich. Gegenstand der Erfindung ist auch eine Vernetzerlösung umfassend eine Phosphatlösung oder eine Calciumlösung sowie einen Gehalt an Vernetzer.

Ferner ist ein Gegenstand der Erfindung ein Kit aufweisend einen teilelastischen Formkörper A, insbesondere eine Formulierung eines teilelastischen Formkörpers A, und einen teilelastischen Formkörper B, insbesondere eine Formulierung eines teilelastischen Formkörpers B, die jeweils unabhängig in Form eines dreidimensionalen Körpers, insbesondere als Flächenelement oder mindestens teilweisen Kiefernegativ, vorliegen, wobei (a) der teilelastische Formkörper A umfasst, (a1) mindestens eine Mineralisationsmatrix umfassend mindestens ein Gel, (a2) mindestens ein wasserlösliches Phosphat oder zu wasserlöslichen Phosphat-Ionen hydrolysierbare Phosphate, und (a3) optional mindestens ein 2-Hydroxycarbonsäure mit einem Kohlenwasserstoff an C-2 und optional ein Puffersystem, (a4) optional wasserlösliche Fluoride oder eine Fluoride freisetzende Verbindung, (a5) optional einen Gehalt an Wasser oder ein Gemisch von Wasser und einem organischen Lösemittel,
(b) der teilelastische Formkörper B umfasst, (b1) mindestens eine Mineralisationsmatrix umfassend mindestens ein Gel, (b2) wasserlösliche Calcium-Ionen oder Calcium-Ionen freisetzende Verbindungen, und
(b3) optional mindestens eine 2-Hydroxycarbonsäure mit einem Kohlenwasserstoff an C-2 und optional ein Puffersystem (b4) optional Wasser oder ein Gemisch von Wasser und einem organischen Lösemittel.

Kit umfassend (I) einen Formkörper in Form eines Flächenelements mit mindestens zwei optional durch eine Membran getrennte Mineralisationsmatrices jeweils unabhängig in Form eines Flächenelements enthaltend das Gel mit einer Schichtdicke von 50 bis 6000 µm, oder (II) zwei separate Formkörper jeweils unabhängig in Form eines Flächenelements mit mindestens jeweils einer Mineralisationsmatrix in Form eines Flächenelements enthaltend das Gel, wobei jeder Formkörper jeweils unabhängig eine Schichtdicke von 10 bis 3000 µm aufweist, wobei der erste Formkörper umfassend Phosphate oder zu wasserlöslichen Phosphat-Ionen hydrolysierbare Phosphate eine Schichtdicke von 50 bis 3000 µm und der zweite Formkörper umfassend Calcium-Ionen oder Calcium-Ionen freisetzende Verbindungen eine Schichtdicke von 10 bis 3000 µm aufweist. In der Alternative des Kiefernegativs umfasst der Formkörper vorzugsweise innere an den Zähnen anliegende Schichten der mindestens einen oder zwei Mineralisationsmatrices. Vorteilhaft kann der Formkörper auch in Form eines mindestens teilweisen Kiefernegativs des Ober- und/oder Unterkiefers vorliegen.

Ferner umfasst das Kit vorzugsweise eine Formulierung in Form a) einer wässrigen Vorbehandlungslösung synonym zu Mundspüllösung umfassend Wasser, 0.1 bis 30 Gew.-% eines gut wasserlöslichen Calcium-Salzes, insbesondere 5 bis 15 Gew.-% in Bezug auf die Gesamtzusammensetzung, vorzugsweise Calciumlactat, Calciumchlorid, Calciumgluconat, Calciumlacto-Gluconat, ein Hydrat der Salze oder eine Mischung enthaltend zwei der Salze, optional einen Gehalt eines Puffersystems, optional Maskierungsmittel oder Aromatisierungsmittel und aufweisend einen pH-Wert von 5.0 bis 12.0, oder die Formulierung in Form b) umfasst mindestens ein gut wasserlösliches Calcium-Salz, umfassend vorzugsweise Calciumlactat, Calciumchlorid, Calciumgluconat, Calciumlacto-Gluconat, ein Hydrat der Salze oder eine Mischung enthaltend zwei der Salze, optional einen Gehalt eines Puffersystems, optional einen Gehalt an Maskierungsmittel oder Aromatisierungsmittel sowie üblichen Formulierungshilfsmitteln, wie Sprengmittel, HPMC etc., insbesondere in Form eines wasserlöslichen Granulats, wasserlöslichen Pellets, Tabletten, als Sachet, Pulver bzw. Granulat in einem Sachet oder löslichen Kapseln. Gegenstand der Erfindung ist auch die Verwendung einer einzelnen Formulierung der Form b) zusammen mit einem Kit umfassend die erfindungsgemässen Formulierungen.

Die Zusammensetzung des Kits ist im Folgenden beschrieben. Die Mundspülung oder Vorbehandlungslösung ist zusammengesetzt aus 0.1 Gew.-% bis 30 Gew.-% eines gut wasserlöslichen Calcium-Salzes, bevorzugt 5 Gew.-% bis 15 Gew.-% Calciumchlorid oder Calciumlactat oder Calciumgluconat oder Calciumlacto-Gluconat oder anderer ausreichend lösliche Calciumsalze. Die Lösung wird mit einem geeigneten Puffer auf einen pH-Wert zwischen 5.0 und 12.0 eingestellt, bevorzugt 8,0 bis 10,0. Zur Geschmacksverbesserung ist eine Aromatisierung oder auch eine Komplexierung von geschmacksbeeinträchtigenden Verbindungen möglich, solange die Apatitabscheidung nicht beeinträchtig wird. Als Puffer eignen sich alle Puffer die in diesem pH-Wert-Bereich gute Pufferkapazität aufweisen z.B. EDTA, Tris, HEPES oder Barbital-Acetat-Puffer aber auch andere Puffersysteme, bevorzug ist Tris.

Weiter bevorzugt ist es, wenn die Formulierungen oder Mundspüllösungen mindestens ein Puffersystem umfasst. Besonders bevorzugte Salze der vorgenannten Säuren sind die Alkali-, Erdalkali- und/oder Zink-Salze der Citronensäure, Aepfelsäure, Weinsäure und/oder Milchsäure oder Tris. Besonders bevorzugte Salze umfassen die Kationen von Natrium, Kalium, Magnesium und/oder Zink der vorgenannten Carboxylate.

Die erfindungsgemässen Formulierungen können vorzugsweise zur Behandlung empfindlicher Zähne, empfindlicher Zahnhälse, säureerodierter Zähne, rissiger Zähne, oberflächliche abradierter Zähne, freiliegender Zahnhälse, gebleichter Zähne, Zähnen nach einer Behandlung kariöser Zahnbereiche ein- (once-a-day) bis zweifach (bspw. one-day-mineralisation) angewendet werden, um eine 2 bis grösser gleich 5 µm dicke, vorzugsweise homogene und im Wesentlichen kristalline Apatitschicht auf den behandelten Oberflächen auszubilden. Grundsätzlich kann die Formulierung bei Bedarf häufiger angewendet werden, beispielsweise nach definierten Zeiträumen.

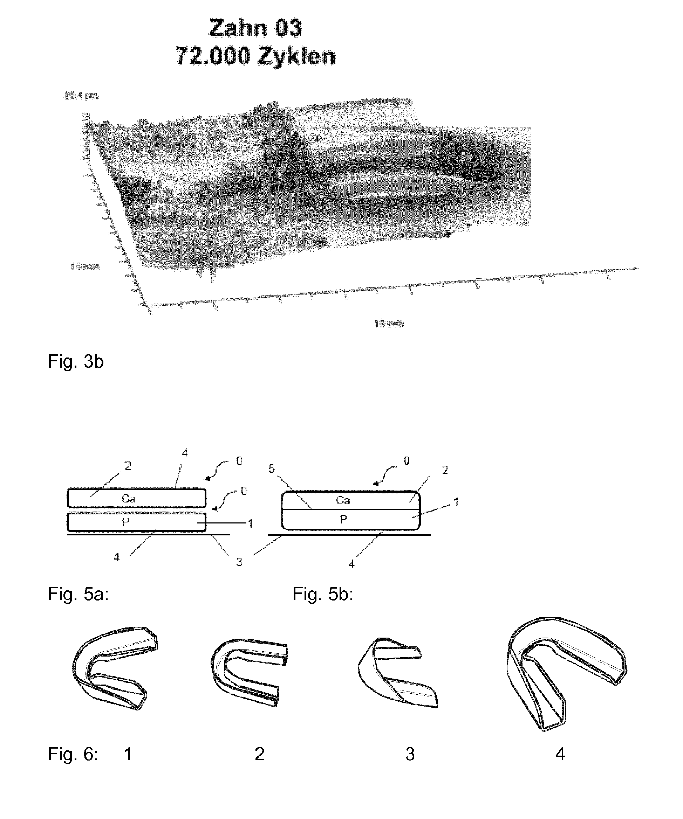

Die Figuren 5a und 5b offenbaren allgemeine Ausführungsformen der erfindungsgemässen Formkörper. Figur 5a zeigt zwei Formkörper 0, wobei die Mineralisationsmatrix 2 die Calcium-Komponente und die Mineralisationsmatrix 1 die Phosphat-Komponente umfasst. Die Hülle (Vernetzung, Beschichtung) wird dargestellt durch 4 und die optionale Membran(schicht) durch 5. In Figur 5a sind zwei Formkörper mit je einer Mineralisationsmatrix und in Figur 5b ein Formkörper mit zwei Mineralisationsmatrices in einer Hülle dargestellt. Die Mineralisationsmatrices können auch jeweils Calcium unterschiedlicher Konzentration enthalten bzw. entsprechend für Phosphat.

Die Erfindung wird anhand der nachfolgenden Beispiele und Figuren näher erläutert, ohne sie auf diese Beispiele zu beschränken.

**Beispiel 1:** Für die phosphationenhaltige Komponente A wird eine Lösung hergestellt, die 29,5 g NaH₂PO₄, 33 g Olaflur, und 27,0 g Milchsäure enthält. Der pH-Wert wird mit 5 N Natronlauge auf 5,4 eingestellt und die Lösung wird mit entionisiertem Wasser auf 250 ml aufgefüllt. 24 ml der Lösung werden mit 6 g Glycerin und 10 g einer 300 Bloom Schweineschwarte-Gelatine unter Erwärmen zu einer dickflüssigen Lösung verarbeitet. In eine Schablone mit der Wandstärke von 500 µm wird etwas Flüssigkeit eingebracht und unter 2 bar Druck gepresst. Nach Verfestigung werden die Streifen der Schablone entnommen und in 1x1 cm grosse Quadrate geschnitten.

Für die calciumionenhaltige Komponente B wird eine Calciumchloridlösung angesetzt, die 29,4 g Calciumchlorid-Dihydrat und 6,3 g Milchsäure enthält. Mit 5 N Natronlauge wird ein pH-Wert von 4.0 eingestellt. Die Lösung wird mit entionisiertem Wasser auf 200 ml aufgefüllt. Zur Herstellung des Gels werden 21,6 g der Lösung mit 8,24 g Glycerin und 8 g Calciumsulfat und 13,6 g 300 Bloom-Gelatine vermischt und erwärmt. Das flüssige Gel wird mit einer Rakel auf eine Dicke von 1 mm ausgestrichen oder in einer Schablone mit der Wandstärke von 1 mm gepresst. Nach Verfestigung werden die Streifen in 1x1 cm grosse Quadrate geschnitten. Für die Vorbehandlungslösung (Mundspüllösung) wird eine 1 molare Calciumchloridlösung mit 0.1 mol Tris-Puffer versetzt und auf den pH-Wert von 9,0 eingestellt.

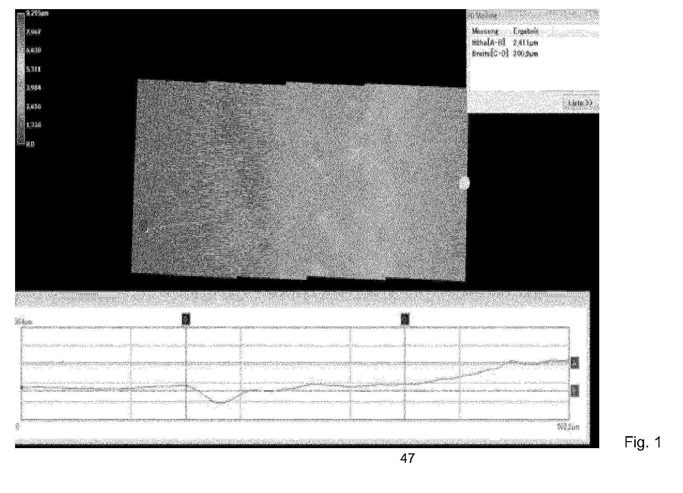

Zur Bewertung der Mineralisationsaktivität werden je 6 Zahnscheiben für 10 s mit 1 M HCl angeäzt, mit der Vorbehandlungslösung abgespült und mit je einem Stück Phosphat-Gel und einem Stück Calciumgel bedeckt. Um die morphologische Änderung der Zahnoberfläche deutlich zu machen, wurde eine Scheibe zuvor zur Hälfte abgeklebt, so dass sie nur hälftig remineralisieren kann. Die Proben werden im Klimaschrank bei 37°C und 95% Luftfeuchtigkeit aufbewahrt und nach 8 bis 16 Stunden mit lauwarmen Wasser und einer weichen Zahnbürste gereinigt. Nach nur einer Behandlung ist die Zahnoberfläche zum grössten Teil mit einer fest haftenden Schicht überzogen. Abbildung 1 zeigt die typische Oberflächenmorphologie der Beschichtung nach einer Behandlung im Grenzbereich zwischen beschichteter und unbeschichteter Probe. Die Schicht kann bis zu 2.5 µm dick sein.

Es wurde gefunden, dass die Apatitschichten im Vergleich zu anderen Carbonsäuren besser wachsen, wenn eine Formulierung enthaltend Milchsäure genutzt wird.

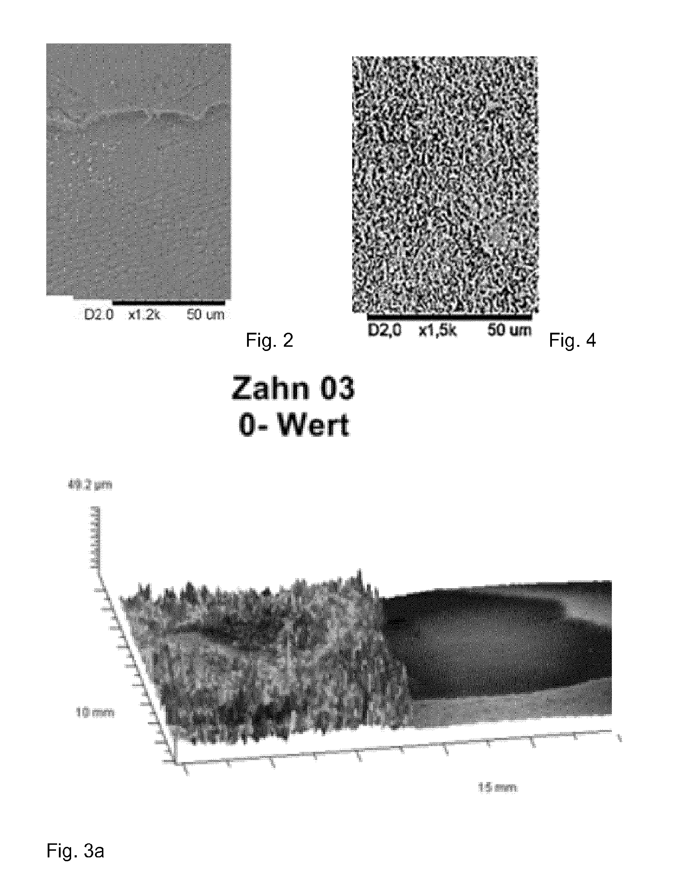

Figur 1: Typische Schichten nach einer Anwendung des Mineralisations-Kits 1: Lichtmikroskopische Aufnahme in Falschfarben, 3D-Mikroskop von Keyence, typische Schicht nach einer Anwendung des Mineralisations-Kits. Die Farben repräsentieren die unterschiedlichen Höhen. Aus dem Linescan lässt sich die Schichtdicke bestimmen. Links: unbehandeltes Dentin (blau) rechts: behandeltes Dentin (grün-rot). Die Kanalstruktur verschwindet unter einer dichten Schicht (3-D-Mikrsokop, Keyence), die Schicht kann bis zu 2.5 µm dick sein. Figur 2: REM-Aufnahme der Grenzfläche Schmelz beschichtet - unbeschichtet).

**Beispiel 2:** Die Herstellung der Komponenten erfolgt wie unter Beispiel 1 beschrieben. Allerdings wurden neben der (Beispiel 2i) Milchsäure als Säurekomponente des Puffersystems auch (Beispiel 2a) Äpfelsäure, (Beispiel 2b) Zitronensäure, (Beispiel 2c) Weinsäure, (Beispiel 2d) Ascorbinsäure, (Beispiel 2e) Trimellitsäure, (Beispiel 2f) Glycolsäure, (Beispiel 2g) Salicylsäure und (Beispiel 2h) Tropasäure verwendet. Sofern die Säuren ausreichend löslich waren, wurde die Calciumlösung auf pH 4.0 und die Phosphatlösung auf pH 5.2 eingestellt.

Nach der Anwendung der Gele wurde über einen Färbetest geprüft, ob es zu einer das Dentin abdichtenden Wirkung gekommen ist. Waren die Säuren unter den nötigen Bedingungen nicht löslich, wurden sie nicht weiter verfolgt.

Prinzip Färbetest: Die Proben werden auf beschichteter und unbeschichteter Seite nach Anfärbung mit 1%iger Rhodaminlösung farbmetrisch mit einem Zwei-Kanalspektrometer Spectraflash 600 Plus mit dem CIE L*a*b*-System mit der Blende 3 mm X4SAV vermessen. Der Unterschied der Farbe wird im Wert delta E angegeben (Farbabstand).

Im Ca-Gel wurde Tris-Puffer (pH-Wert: 7,2, bis 9,0) eingesetzt. Im P-Gel wurde ein Natrium-Acetat Puffer eingesetzt (pH-Wert: 3,7 bis 5,7)

Tabelle 1 fasst die Ergebnisse zusammen. Demnach sind neben der den Calciumsalzen der Milchsäure auch die der Säuren Äpfelsäure, Asparaginsäure, Ascorbinsäure und Glycolsäure ausreichend löslich und zeigen eine Abscheidung nach Anwendung des Kits. Allerdings führt die Ascorbinsäure zu einer starken Gelbverfärbung der Zähne und scheidet damit für die Anwendung aus, die anderen Säuren führten zu weniger guten Ergebnissen als die Milchsäure. Generell sind zudem gelb gefärbte Gele für die Akzeptanz beim Anwender unerwünscht. Aus diesem Grund sind die Glycolsäure, Asparaginsäure und die Äpfelsäure als alleinige Säuren nicht in der Anwendung bevorzugt. Eine Verwendung in Kombination mit Milchsäure oder Milchsäurederivaten ist möglich.

**Tabelle 1: 2-Hydroxycarbonsäuren der Beispiele 2a bis 2i.**

| Carbonsäuren | VB-Lösung | Ca-Gel | P-Gel | Funktionstest (delta E) | pKs |
|---|---|---|---|---|---|
| Äpfelsäure | pH 8,5 Ausfällung | 0,034 mol = 4,6 g Ausfällung | 0,06 mol = 8,04 g gelblich-trüb bei pH 5,25 | 42,41 ± 5,39 | 3,46/5,10 |
| | pH 9,02 klare Lösung | 0,017 mol = 2,3 g klare Lösung, nach 24h Ausfällung | 0,03 mol = 4,02 g klare gelbe Lösung, nach 24 Flockenbildung | | |
| | pH 9,53 klare Lösung | | | | |
| Zitronensäure | pH 9,00 klare Lösung | 0,035 mol = 6,67 g Ausfällung | 0,06 mol = 11,52 g gelblich-trüb bei pH 5,21 | | 3,13/4,76/6,40 |

| Weinsäure | nicht hergestellt | nicht hergestellt | Zugabe von Weinsäure erfolgt sofortige weiße Ausfällung | | 2,98/4,34 |
|---|---|---|---|---|---|
| Ascorbinsäure | pH 9,00 klare Lösung, verfärbt sich gelb | 0,034 mol = 5,99 g klar, verfärbt sich gelb | 0,06mol = 10,57 g klar, verfärbt sich Immer gelber | 56,64 ± 9,85 gelb verfärbter Zahn | 4,37 ±0,53 |
| Asparaginsäure | unlöslich | 0,034 mol = 4,99 g klar (nach Zugabe von NaOH) | 0,06 mol = 7,99 g trüb, leicht gelblich, klar nach Zugabe von NaOH | 45,96 ±2,63 | 1,99/3,99/"9,9 0' |
| Trimellitsäure | nicht hergestellt | 0,03 mol = 3 g massive Fällung | 0,06 mol = 6.6 g im Warmen löslich ab pH 4.2 | 42,05 ± 11,24 mit Essigsäure-Ca-Gel kombiniert | k.A. |
| Glycolsäure | nicht hergestellt | 0,034 mol = 3,16 g nach Zugabe von NaOH gelartige Fällung | 0,06 mol = 6,37 g klare, gelbe Lösung | 35,32 ±4,19 mit Essigsäure-Ca- Gel kombiniert | 3,83 |
| Salicylsäure | nicht hergestellt | unlöslich | | | 2,75/12,38 |
| Tropasäure | unlöslich | | | | |
| Milchsäure | pH 9,00 klare Lösung | 0,034 mol = 3,15 g klar | 0,06 mol = 5,40 g klar | 49,83 ±4,02 | 3,90 |

In zusätzlichen Anwendungstests, die auch mit Essigsäure hergestellte Formulierungen umfasste, hat sich gezeigt, dass die Formulierungen der Milchsäure besser verträglich waren. Dies wird zum Teil auch auf die Geruchsentwicklung von Essigsäure oder anderer geruchsintensiven Säuren zurückgeführt, so dass die hergestellten Formulierungen als unangenehmer wahrgenommen werden.

Die Verträglichkeit der Carbonsäuren ist für eine Anwendung wesentlich, denn die Formulierungen sollen über einige Stunden im Mund verbleiben und zu keinen Hautoder Schleimhautirritationen führen. Ein weiterer Vorteil der Milchsäure in den erfindungsgemäßen Formulierungen ist ihre konservierende Wirkung. Um eine konservierende Wirkung zu entfalten ist bei schwachen Säuren deren pH-Wert und ein saurer pH-Wert in der Formulierung notwendig, da nur undissoziierte Moleküle durch die Zellmembran in das Innere von Mikroorganismen eindringen können.

Erfindungsgemäss ist es daher besonders bevorzugt, wenn die Milchsäure in der Phosphat-Komponente und vorzugsweise in der Calcium-Komponente eingesetzt wird.

**Beispiel 3:** Die Herstellung der Komponenten erfolgt wie unter Beispiel 1 beschrieben.

Zur Bewertung der Mineralisationsaktivität werden je 6 Zahnscheiben der Vorbehandlungslösung abgespült und mit je einem Stück Phosphat-Gel und einem Stück Kalziumgel bedeckt. Um die morphologische Änderung der Zahnoberfläche deutlich zu machen, wurde eine Scheibe zuvor zur Hälfte abgeklebt, so dass sie nur hälftig remineralisieren kann. Die Proben werden im Klimaschrank bei 37°C und 95% Luftfeuchtigkeit aufbewahrt. Täglich gewachsen und einer erneuten Gelbehandlung unterzogen. Nach drei Behandlungen war die Zahnoberfläche so gut wie vollständig mit einer fest anhaftenden Schicht überzogen.

Die schmelzähnliche Stabilität lässt sich durch Zahnbürstabrasionsuntersuchungen zeigen. Nach 72.000 homogenen Bürstenstrichen (Belastung 150 g) auf einer Zahnprobe die halbseitig behandelt wurde, zeigt sich, dass das ungeschützte Dentin deutlich stärker abradiert wird als die ungeschützte Seite die ähnlich dem nativen Schmelz kaum abradiert wird. Figuren 3a und 3b: 3x halbseitig behandelte Zahnscheiben vor der Zahnbürstabrasion (links) und nach der Zahnbürstabrasion. Es ist gut zu erkennen, dass die Behandlung mit dem Mineralisations-Kit das Dentin vor Abrasion schützt, das nicht behandelte Dentin wird stark abradiert, zu erkennen in einer 3D-Darstellung von biomimetisch behandelten Zähnen auf denen Fluorapatit abgeschieden wurde und nicht behandelten Zähnen, deren Zahnflächen anschließend beide mittels Zahnbürste abradiert wurde. Die unbehandelten Zähne sind stark abradiert.

**Beispiel 4:** Zur Herstellung der P-Komponente werden für die P-Lösung 5,9 g Na₂HPO₄, 9,1g Milchsäure, 6,6 g Olaflur und 0.6 g 5 M NaOH auf 50 ml mit entionisiertem Wasser aufgefüllt. Die Herstellung des Gels erfolgt wie unter Beispiel 1 beschrieben. Die Calciumlösung für die Ca-Komponente wird durch Lösen in entionisiertem Wasser von 14,7 g CaCl₂, 3,15 g Milchsäure, 10 g 5 m Natronlauge in insgesamt 100 ml Lösung hergestellt. Die Herstellung des Gels entspricht der Beschreibung in Beispiel 1. Das Gleiche gilt für die Vorbehandlungslösung.

Die Vernetzung wird aber bei beiden Gelen für jeweils 2x20s von beiden Seiten durchgeführt. Dabei liegt die GDA-Konzentration in der Ca-Vernetzerlösung bei 0.5 Gew.-%. Das P-Gel für 30 s mit einer 0.375%igen GDA-Lösung behandelt, die durch Mischen der P-Lösung mit der entsprechenden Menge GDA hergestellt wird. Die Gelstreifen werden im Anschluss nur trocken getupft.

Zur Bewertung der Mineralisationsaktivität werden je 6 Zahnscheiben für 10 s mit 1 M HCl angeäzt, mit der Vorbehandlungslösung abgespült und mit je einem Stück Phosphat-Gel und einem Stück Calziumgel bedeckt. Um die morphologische Änderung der Zahnoberfläche deutlich zu machen, wurde eine Scheibe zuvor zur Hälfte abgeklebt, so dass sie nur hälftig remineralisieren kann. Die Proben werden im Klimaschrank bei 37°C und 95 % Luftfeuchtigkeit aufbewahrt und nach 8 bis 12 Stunden mit lauwarmen Wasser und einer weichen Zahnbürste gereinigt. Nach nur einer Behandlung ist die Zahnoberfläche zum grössten Teil mit einer fest haftenden Schicht überzogen.

Im Elektronenmikroskop ist eine weitgehend homogene Schicht aus kleinen nadeligen Kristallen zu erkennen.

Fig. 4: Bewachsene Dentinoberfläche. Die porenreiche Dentinfläche ist von einer gleichmässigen Schicht nadeliger Kristalle überwachsen. Nach Behandlung mit dem Mineralisations-Kit (doppelseitige GDA-Vernetzung).

Figur 5a, 5b: Zwei Formkörper mit je einer Mineralisationsmatrix in Figur 5a und in Figur 5b ein Formkörper mit zwei Mineralisationsmatrices in einer Hülle.

Figur 6: Zeigt drei Komponenten: 1. Schutzschiene (Applikations-Zahnschiene), 2. Calcium-Komponente (erster Formkörper I), 3. Phosphat-Komponente (weiterer Formkörper II). 1 und 2 kann zu einer Form (Applikations-Zahnschiene und der erste Formkörper bilden eine Einheit) kombiniert werden, 3 (weiterer Formkörper) darf erst kurz vor Beginn der Mineralisation zugefügt werden.

## Patentansprüche

1. Formulierung geeignet zur Abscheidung von Apatit ausgewählt aus Fluorapatit und Hydroxylapatit oder deren Gemischen auf Zähnen von Wirbeltieren,
**dadurch gekennzeichnet, dass**
- die Formulierung mindestens einen teilelastischen Formkörper aufweist, umfassend mindestens eine Mineralisationsmatrix enthaltend mindestens ein Gel, wobei das Gel mindestens eine Carbonsäure oder eine Mischung von Carbonsäuren ausgewählt aus an C-2 mit einem Kohlenwasserstoff-Rest substituierten 2-Hydroxycarbonsäuren aufweist, insbesondere Milchsäure, und optional ein Puffersystem, und
a)die mindestens eine Mineralisationsmatrix ein Gel enthaltend wasserlösliche Phosphate oder zu wasserlöslichen Phosphat-Ionen hydrolysierbare Phosphate und einen pH-Wert von 2 bis 8 aufweist, und
b)die mindestens eine oder eine zweite Mineralisationsmatrix ein zweites Gel umfassend Calcium-Ionen oder Calcium-Ionen freisetzende Verbindungen und einen pH-Wert von 3.5 bis 14 aufweist.

2. Formulierung nach Anspruch 1, **dadurch gekennzeichnet, dass** der Formkörper zumindest teilweise in mindestens einer Ebene eine gegenüber wässrigen Medien verminderte Löslichkeit im Vergleich zur Mineralisationsmatrix aufweist, wobei die Ebene als Membran fungiert.

3. Formulierung nach Anspruch 1 oder 2, **dadurch gekennzeichnet, dass** die mindestens eine Mineralisationsmatrix in einem ersten Formkörper I. und die zweite Mineralisationsmatrix in einem zweiten Formkörper II. vorliegen.

4. Formulierung nach einem der Ansprüche 1 bis 3, **dadurch gekennzeichnet, dass** in a) die mindestens eine Mineralisationsmatrix ein Gel aufweist, umfassend
(i) wasserlösliche Phosphate oder zu wasserlöslichen Phosphat-Ionen hydrolysierbare Phosphate,
(ii) einen Gehalt an Wasser oder ein Gemisch von Wasser und einem organischen Lösemittel,
(iii) mindestens eine Carbonsäure oder eine Mischung von Carbonsäuren, insbesondere umfassend Milchsäure, und optional ein Puffersystem,
und/oder die zweite Mineralisationsmatrix oder die mindestens eine Mineralisationsmatrix in (b) ein zweites Gel aufweist, umfassend
(i) Calcium-Ionen oder Calcium-Ionen freisetzende Verbindungen,
(ii) optional Wasser oder ein Gemisch von Wasser und einem organischen Lösemittel, und
(iii) mindestens eine Carbonsäure oder eine Mischung von Carbonsäuren, insbesondere umfassend Milchsäure, und optional ein Puffersystem.

5. Formulierung nach einem der Ansprüche 1 bis 4, **dadurch gekennzeichnet, dass** die mindestens eine Mineralisationsmatrix ein Gel aufweist, umfassend mindestens ein wasserlösliches Fluorid oder eine Fluoride freisetzende Verbindung, optional umfassend mindestens eine unsubstituierte oder substituierte Alkyl-Gruppe aufweisende quaternäre mono- oder poly-Ammonium-Verbindung enthält, wie N,N,N'-Tris(2-hydroxyethyl)-N'-octadecyl-1,3-diaminopropandihydrofluorid (Olaflur), Aminfluoride, wie Oleaflur, Decaflur, Ethanolamin-Hydrofluorid und/oder wasserlösliche anorganische Fluoride, wie Alkalifluoride, Natriumfluorid, Kaliumfluorid, Zinnfluorid, Ammoniumfluorid oder Fluoride freisetzende anorganische Fluoride, wie Zinkfluorid, Zinkhydroxyfluorid.

6. Formulierung nach einem der Ansprüche 1 bis 5, **dadurch gekennzeichnet, dass** das Gel mindestens einen Gelbilder ausgewählt aus denaturiertem Kollagen, Hydrokolloiden, Polypeptiden, Proteinhydrolysaten, Polysaccariden, Polyacrylaten oder Mischungen umfassend mindestens zwei der genannten Gelbilder enthält, wobei das Gel vorzugsweise Gelatine und ein Polyol, vorzugsweise Glycerin, deren Addukte und/oder deren Umsetzungsprodukte umfasst.

7. Formulierung nach einem der Ansprüche 1 bis 6, **dadurch gekennzeichnet, dass**
a) die mindestens eine Mineralisationsmatrix umfassend mindestens ein Gel in mindestens einem teilelastischen Formkörper in Form eines Flächenelements oder mindestens teilweisen Kiefernegativs vorliegt, wobei der Formkörper insbesondere mindestens zwei an der äusseren Oberfläche angeordnete Ebenen oder zumindest eine teilweise äussere Hülle aufweist, die eine gegenüber wässrigen Medien verminderte Löslichkeit als die Mineralisationsmatrix aufweisen, und/oder
b) die mindestens eine oder zwei Mineralisationsmatrices umfassend mindestens ein Gel in mindestens einem teilelastischen Formkörper in Form eines Flächenelements oder mindestens teilweisen Kiefernegativs vorliegen.

8. Formulierung nach einem der Ansprüche 1 bis 7, **dadurch gekennzeichnet, dass** die Ebenen oder die Hülle die äussere Begrenzung der Mineralisationsmatrix bilden.

9. Formulierung nach einem der Ansprüche 1 bis 8, **dadurch gekennzeichnet, dass** sie umfasst, zwei separate Formkörper jeweils unabhängig in Form eines Flächenelements mit mindestens jeweils einer Mineralisationsmatrix enthaltend ein Gel, wobei jeder Formkörper jeweils unabhängig eine Schichtdicke von 10 bis 3000 µm aufweist, wobei der erste Formkörper umfassend Phosphate oder zu wasserlöslichen Phosphat-Ionen hydrolysierbare Phosphate eine Schichtdicke von 50 bis 3000 µm und/oder der zweite Formkörper umfassend Calcium-Ionen oder Calcium-Ionen freisetzende Verbindungen eine Schichtdicke von 10 bis 3000 µm aufweist.

10. Verfahren zur Herstellung einer Formulierung, insbesondere nach einem der Ansprüche 1 bis 9, geeignet zur biomimetischen Abscheidung von Apatit ausgewählt aus Fluorapatit, Hydroxylapatit oder deren Gemischen auf Zähnen von Wirbeltieren indem,
(1) mindestens ein teilelastischer Formkörper umfassend mindestens eine Mineralisationsmatrix enthaltend mindestens ein Gel mit wasserlöslichen Phosphaten oder zu wasserlöslichen Phosphat-Ionen hydrolysierbare Phosphate hergestellt wird, und der Formkörper hergestellt wird, indem
a)zur Herstellung mindestens einer Mineralisationsmatrix enthaltend das Gel in einem ersten Schritt eine Mischung von
(i) 0.05 bis 4 mol/l, 0.5 bis 1.5 mol/l wasserlöslichen Phosphaten oder zu wasserlöslichen Phosphat-Ionen hydrolysierbaren Phosphaten,
(ii) einer entsprechenden Menge Wasser oder einem Gemisch von Wasser und einem organischen Lösemittel,
(iii) mindestens eine an C-2 mit einem Kohlenwasserstoff subsituierte 2-Hydroxycarbonsäure und optional ein Puffersystem
(iv) 0 bis 6000 Gew.-ppm wasserlöslichem Fluorid oder einer Fluoride freisetzenden Verbindung, gemischt werden,
in einem weiteren Schritt wird aus der in a) hergestellten Mischung
b)mit Gelatine und optional Glycerin unter Erwärmen das Gel hergestellt,
c) Formung des Gels unter Ausbildung der Mineralisationsmatrix, optional Verfestigung.

11. Verfahren zur Herstellung einer Formulierung, insbesondere nach einem der Ansprüche 1 bis 13, geeignet zur biomimetischen Abscheidung von Apatit ausgewählt aus Fluorapatit, Hydroxylapatit oder deren Gemischen auf Zähnen von Wirbeltieren indem,
(1) mindestens ein teilelastischer Formkörper umfassend mindestens eine Mineralisationsmatrix enthaltend mindestens ein Gel mit Calcium-Ionen oder Calcium-Ionen freisetzende Verbindungen hergestellt wird, und der Formkörper hergestellt wird, indem
a)zur Herstellung mindestens einer Mineralisationsmatrix enthaltend das Gel in einem ersten Schritt eine Mischung von
(i) 0.1 bis 2 mol/l Calcium-Ionen oder Calcium-Ionen freisetzenden Verbindungen,
(ii) einer entsprechenden Menge Wasser oder einem Gemisch von Wasser und einem organischen Lösemittel,
(iii) mindestens eine an C-2 mit einem Kohlenwasserstoff subsituierte 2-Hydroxy carbonsäure und optional ein Puffersystem gemischt werden,
in einem weiteren Schritt wird aus der in a) hergestellten Mischung
b)mit Gelatine und optional Glycerin unter Erwärmen das Gel hergestellt,
c) Formung des Gels unter Ausbildung der Mineralisationsmatrix, optional Verfestigung.

12. Verfahren nach Anspruch 10 oder 11 **dadurch gekennzeichnet, dass** der Formkörper in (1) zumindest teilweise in mindestens einer Ebene, eine gegenüber wässrigen Medien verminderte Löslichkeit als die Mineralisationsmatrix aufweist, und der Formkörper hergestellt wird,
indem d) eine an einer äusseren Oberfläche angeordnete Ebene der mindestens einen Mineralisationsmatrix ausgebildet wird unter Bildung des Formkörpers.

13. Verfahren nach einem der Ansprüche 10 bis 12, **dadurch gekennzeichnet, dass** das im weiteren Schritt b) hergestellte Gel geformt wird, insbesondere zu einem Flächenelement oder einer individuellen dreidimensionalen Form, und das Gel in dieser Form verfestigbar ist.

14. Formkörper der Formulierung erhältlich nach einem Verfahren nach einem der Ansprüche 10 oder 11 bis 13.

15. Kit umfassend mindestens eine Formulierung aufweisend einen teilelastischen Formkörper A und einen separaten teilelastischen Formkörper B, jeweils unabhängig umfassend eine Formulierung nach einem der Ansprüche 1 bis 9 oder 14 oder hergestellt nach einem der Ansprüche 10 bis 13, wobei
(a) der teilelastische Formkörper A umfasst
(a1) mindestens eine Mineralisationsmatrix umfassend mindestens ein Gel,
(a2) mindestens ein wasserlösliches Phosphat oder zu wasserlöslichen Phosphat-Ionen hydrolysierbare Phosphate, und
(a3) mindestens eine an C-2 mit einem Kohlenwasserstoff substituierte 2-Hydroxycarbonsäure und optional ein Puffersystem
(a4) optional wasserlösliche Fluoride oder eine Fluoride freisetzende Verbindung
(a5) optional einen Gehalt an Wasser oder ein Gemisch von Wasser und einem organischen Lösemittel
(b) der teilelastische Formkörper B umfasst
(b1) mindestens eine Mineralisationsmatrix umfassend mindestens ein Gel,
(b2) wasserlösliche Calcium-Ionen oder Calcium-Ionen freisetzende Verbindungen, und
(b3) mindestens eine an C-2 mit einem Kohlenwasserstoff substituierte 2-Hydroxycarbonsäure und optional ein Puffersystem
(b4) optional Wasser oder ein Gemisch von Wasser und einem organischen Lösemittel, oder
einen teilelastischen Formkörper C umfassend eine Formulierung, wobei der
(c) teilelastische Formkörper C umfasst
(c1) mindestens eine Mineralisationsmatrix umfassend mindestens ein Gel,
(c1.1) mindestens ein wasserlösliches Phosphat oder zu wasserlöslichen Phosphat-Ionen hydrolysierbares Phosphat, und
(c1.2) mindestens eine an C-2 mit einem Kohlenwasserstoff substituierte 2-Hydroxycarbonsäure und optional ein Puffersystem
(c1.3) optional wasserlösliche Fluoride oder eine Fluoride freisetzende Verbindung
(c1.4) optional einen Gehalt an Wasser oder ein Gemisch von Wasser und einem organischen Lösemittel
(c2) optional eine Membran(schicht)
(c3) mindestens eine Mineralisationsmatrix umfassend mindestens ein Gel,
(c3.1) wasserlösliche Calcium-Ionen oder Calcium-Ionen freisetzende Verbindungen, und
(c3.2) mindestens eine an C-2 mit einem Kohlenwasserstoff substituierte 2-Hydroxycarbonsäure und optional ein Puffersystem
(c3.3) optional Wasser oder ein Gemisch von Wasser und einem organischen Lösemittel, wobei der teilelastische Formkörper C den Schichtaufbau c1 und c3 oder c1, c2 und c3 aufweist.
